# EUROPEAN PATENT SPECIFICATION

(11) **EP 3 984 465 B1**
(45) Date of publication and mention of the grant of the patent: **09.12.2026**
(21) Application number: 21213125.4
(22) Date of filing: 01.07.2014
(51) Int. Cl.: A61B 8/00, A61B 8/08, G16H 30/20, G16H 30/40

(54) **SHARING INFORMATION OF MEDICAL IMAGING APPARATUS**
GEMEINSAME NUTZUNG DER INFORMATIONEN EINER MEDIZINISCHEN BILDGEBUNGSVORRICHTUNG
PARTAGE DES INFORMATIONS D'APPAREILS D'IMAGERIE MÉDICALE

(30) Priority: 01.07.2013 KR 20130076594; 25.06.2014 KR 20140078390
(43) Date of publication of application: 20.04.2022
(62) Document number(s) of the earlier application(s) in accordance with Art. 76 EPC: 17193400.3 / 3 298 966; 14175277.4 / 2 821 014
(73) Proprietor: Samsung Electronics Co., Ltd., Suwon-si, Gyeonggi-do 16677 (KR)
(72) Inventor: Jung, Jong-woo, Neung-dong, Hwaseong-si (KR); Yang, Eun-ho, Seoul (KR)
(74) Representative: Gulde & Partner

(56) References cited:
- US-A- 5 715 823
- US-A1- 2002 173 721
- US-A1- 2004 204 649
- US-A1- 2005 043 620
- US-A1- 2008 039 722
- US-B1- 6 475 146

## Description

The present disclosure relates to a method of sharing information about ultrasound images between an ultrasound apparatus and an external device, to a communication method of a medical expert device, to a non-transitory computer-readable recording medium, to an ultrasound apparatus and to a medical expert device.

An ultrasound diagnosis apparatus transfers ultrasound signals from a body surface of an object toward a predetermined portion inside the object to obtain a tomogram image of a soft tissue or an image about blood flow by using information of the ultrasound signal reflected from organs inside the object.

Such an ultrasound diagnosis apparatus is small, cheap, and capable of displaying images in real-time. Also, the ultrasound diagnosis apparatus has high reliability without a risk of X-ray exposure, and thus, is widely used with other imaging diagnosis apparatuses such as an X-ray diagnosis apparatus, a computerized tomography (CT) scanner, a magnetic resonance image (MRI) apparatus, and a nuclear medicine imaging apparatus.

However, there may be a large time difference between a point of time when a sonographer captures an ultrasound image and a point of time when a doctor analyzes the corresponding ultrasound image. Therefore, it takes a long time to diagnose the ultrasound image, and accordingly, an ultrasound image may need to be captured again. Thus, the sonographer, a patient, and a doctor may experience inconvenience. Accordingly, an ultrasound diagnosis system is necessary for allowing a patient or a doctor may immediately identify an ultrasound image when a sonographer captures the ultrasound image.
A system and method for incorporating the functionality of a personal digital assistant ("PDA") with a diagnostic medical ultrasound imaging system is disclosed in US 6,475,146 B1. The PDA is capable of controlling the ultrasound system via a wired or wireless communications link. In addition, the personal information management ("PIM") functions of the PDA are integrated together and with the system control functionality to permit automated control and configuration of the ultrasound system based on data stored by the PIM applications.
The invention is defined by the appended claims. The description that follows is subjected to this limitation. Any disclosure lying outside the scope of said claims is only intended for illustrative as well as comparative purposes.

One or more examples provide an ultrasound apparatus sharing ultrasound information about an ultrasound image with a receiving device according to a sharing level of the receiving device, and an information sharing method of the ultrasound apparatus.

One or more examples also provide a medical expert device capable of sending/receiving information about an ultrasound image to/from an ultrasound apparatus, and a communication method of the medical expert device.

According to an aspect of an example, there is provided a method of sharing information with an external device by an ultrasound apparatus, the method including: acquiring an ultrasound image of an object, preferably by transmitting an ultrasound signal to the object, and receiving an ultrasound response signal from the object; identifying a sharing level of an external device, which is preferably set in advance, for sharing the ultrasound image; and transmitting ultrasound information about the ultrasound image to the external device according to the sharing level.

The ultrasound information may be displayed on the external device in real-time.

The external device may include a first device and a second device, and the transmitting may include: transmitting first ultrasound information corresponding to a first sharing level of the first device to the first device; and transmitting second ultrasound information corresponding to a second sharing level of the second device to the second device. In this case, the identifying a sharing level of an external device comprises identifying a first sharing level of a first device and identifying a second sharing level of a second device.

The first device may include an examinee device, and the second device may include a medical expert device.

The ultrasound information may comprise ultrasound image information for sharing the ultrasound image and additional information in addition to the ultrasound image information.

The ultrasound image information may comprise image data or video data allowing the acquired ultrasound image to be reconstructed in the external device.

The transmitting of the ultrasound information may include generating the ultrasound information by adding at least one annotation related to all or some of the ultrasound image to the ultrasound image.

The adding of the at least one annotation to the ultrasound image may include: analyzing the ultrasound image; recommending at least one annotation related to all or some of the ultrasound image based on a result of the analyzing; and displaying the at least one recommended annotation on the ultrasound image.

The adding of the at least one annotation to the ultrasound image may include: displaying an annotation list that is set in advance; receiving a selection of at least one annotation from the annotation list; and displaying the selected at least one annotation on the ultrasound image.

The displaying of the selected at least one annotation on the ultrasound image may include: receiving a drag & drop input that comprises dragging the selected at least one annotation to a region where the ultrasound image is displayed and dropping the at least one annotation; and displaying the selected at least one annotation on the ultrasound image based on the drag & drop input.

The method may further include: when a touch input of touching the at least one annotation displayed on the ultrasound image for a predetermined length of time or longer is received, activating the touched annotation to be moved; receiving a drag input of the user on the touched at least one annotation; and moving the touched at least one annotation according to the drag input of the user.

The method may further include: providing an activated annotation list about at least one annotation added to the ultrasound image on a predetermined region of a screen.

The transmitting of the ultrasound information may include: selecting a region of interest from the ultrasound image; indicating an identification mark on the region of interest; and transmitting the ultrasound information including information about the region of interest that is indicated by the identification mark to the external device.

The indicating of the identification mark may include: adding a color to the region of interest.

The transmitting of the second ultrasound information may include generating the second ultrasound information including at least one of analysis information about the ultrasound image of the sonographer, measurement information with respect to the ultrasound image, and examinee information.

The method may further include: receiving a message about the ultrasound image from the external device; and displaying the received message on a screen.

The method may further include: providing a chatting window for communicating with the medical expert device on a screen; receiving a user input about the ultrasound image through the chatting window; transmitting the user input to the medical expert device; and receiving a response message to the user input from the medical expert device.

The method may further include: transmitting a request for confirmation about the second ultrasound information to the medical expert device; and receiving a confirmation message of the second ultrasound information from the medical expert device.

The method may further include: receiving control information from the medical expert device; and executing a control command corresponding to the control information.

The control information may include at least one of a control command for selecting and displaying another ultrasound image, a control command for expanding or reducing the ultrasound image, a control command for storing the ultrasound image, a control command for 3-dimensional (3D) rendering the ultrasound image, a control command for adding an annotation or a body marker, a control command for measuring the region of interest, and a control command for correcting the analysis information about the ultrasound image.

The method may further include: providing a list of devices that are capable of communicating with the ultrasound apparatus; and receiving a selection of the external device from the list of devices.

The method may further include: requesting a server for an authentication of the selected external device; and receiving information of a sharing level of the external device from the server, when the authentication has succeeded.

According to another aspect of an example, there is provided a method of communicating with at least one ultrasound apparatus by a medical expert device, the method including: displaying an ultrasound image list including at least one ultrasound image acquired by the at least one ultrasound apparatus on a first region of a screen; receiving a selection of an ultrasound image from the ultrasound image list; displaying the selected ultrasound image on a second region of the screen; and communicating with an ultrasound apparatus that acquires the selected ultrasound image.

The displaying of the ultrasound image list may include displaying the ultrasound list including a real-time ultrasound image transmitted from the at least one ultrasound apparatus and an ultrasound image stored in advance.

The displaying of the ultrasound image list may further include indicating an identification mark on the real-time ultrasound image to distinguish the real-time ultrasound image from the ultrasound image stored in advance.

The displaying of the selected ultrasound image may include displaying at least one of analysis information of a sonographer about the selected ultrasound image, measurement information with respect to the selected ultrasound image, and examinee information, according to a sharing level of the medical expert device.

The displaying of the selected ultrasound image may include displaying a pointer of the ultrasound apparatus and a pointer of the medical expert device on the selected ultrasound image.

The communicating may include: providing a chatting window for communicating with the ultrasound apparatus on a third region of the screen; receiving an input about the selected ultrasound image through the chatting window; and transmitting the received input to the ultrasound apparatus.

The communicating may include: receiving a request for confirmation about the selected ultrasound image from the ultrasound apparatus; receiving confirmation information about the selected ultrasound image; and transmitting the confirmation information to the ultrasound apparatus.

The transmitting of the confirmation information may include: displaying a graphical user interface (GUI) for receiving a confirmation input of the selected ultrasound image; and receiving a confirmation input about the selected ultrasound image through the GUI.

The communicating may include transmitting control information for controlling the ultrasound apparatus to the ultrasound apparatus.

The transmitting of the control information may include transmitting to the ultrasound apparatus at least one of a control command for selecting and displaying another ultrasound image, a control command for expanding or reducing the ultrasound image, a control command for storing the ultrasound image, a control command for 3-dimensional (3D) rendering the ultrasound image, a control command for adding an annotation or a body marker, a control command for measuring the region of interest, and a control command for correcting the analysis information about the ultrasound image.

The method may further include communicating with an examinee device that displays the selected ultrasound image.

The communicating with the examinee device may include: receiving a description by a medical expert about the selected ultrasound image; and transmitting the description by the medical expert to the examinee device.

The description by the medical expert may be displayed on the examinee device in real-time.

According to another aspect of an example, there is provided a method of communicating with an examinee device by a medical expert device, the method including: displaying an ultrasound image of an examinee on a screen of the medical expert device; receiving a description by a medical expert about the ultrasound image; and transmitting the description by the medical expert to the examinee device displaying the ultrasound image, wherein the description by the medical expert is displayed on the examinee device.

The description by the medical expert may be displayed on the examinee device in real-time, or stored in the examinee device.

According to another aspect of an example, there is provided an ultrasound apparatus including: an ultrasound image obtaining unit for acquiring an ultrasound image of an object by transmitting an ultrasound signal to the object and receiving an ultrasound response signal from the object; a control unit for identifying a sharing level of an external device, which is set in advance, for sharing the ultrasound image; and a communication unit for transmitting ultrasound information about the ultrasound image to the external device, according to the sharing level that is set in advance.

The external device may include a first device and a second device, and the control unit generates first ultrasound information corresponding to a first sharing level of the first device, and second ultrasound information corresponding to a second sharing level of the second device.

The ultrasound apparatus may further include an image processor for adding at least one annotation relating to all or some of the ultrasound image to the ultrasound image.

The ultrasound apparatus may further include: a user input unit for receiving a selection of at least one annotation from the annotation list displayed on the screen; and a display unit for displaying the selected at least one annotation on the ultrasound image.

The user input unit may receive a drag & drop input that comprises dragging the selected at least one annotation to a region where the ultrasound image is displayed and dropping the at least one annotation, and the display unit may display the selected at least one annotation on the ultrasound image based on the drag & drop input.

When a touch input of touching the annotation displayed on the ultrasound image for a predetermined length of time or longer is received, the control unit may activate the touched at least one annotation to be moved, the user input unit receives a drag input of the user on the touched at least one annotation, and the display unit may move the touched at least one annotation according to the drag input of the user

The ultrasound apparatus may further include a display unit for providing an activated annotation list about at least one annotation added to the ultrasound image on a predetermined region of the screen.

The control unit may generate the ultrasound information by selecting a region of interest from the ultrasound image and adding a color to the region of interest.

The second ultrasound information may include at least one of analysis information of a sonographer about the ultrasound image, measurement information with respect to the ultrasound apparatus, and examinee information.

The communication unit may receive a message about the ultrasound image from the external device, and the control unit may control the display unit to display the received message on a screen.

The ultrasound apparatus may further include: a display unit for providing a chatting window for communicating with the second device on a screen; and a user input unit for receiving a user input about the ultrasound image through the chatting window, wherein the communication unit may transmit the user input to the second device and receive a response message to the user input from the second device.

The communication unit may transmit a request for confirmation about the second ultrasound information to the second device and receive a confirmation message of the second ultrasound information from the second device.

The communication unit may receive control information from the medical expert device, and the control unit may execute a control command corresponding to the control information.

The ultrasound apparatus may further include: a display unit for providing a list of devices that are capable of communicating with the ultrasound apparatus; and a user input unit for receiving a selection of the external device from the list of devices.

The communication unit may request a server for an authentication of the selected external device, and receive information of a sharing level of the external device from the server, when the authentication has succeeded.

According to another aspect of an example, there is provided a medical expert device including: a display unit for displaying an ultrasound image list including at least one ultrasound image acquired by at least one ultrasound apparatus on a first region of a screen, and displaying a ultrasound image selected from the ultrasound image list on a second region of the screen; a user input unit for receiving a selection of the ultrasound image from the ultrasound image list; a communication unit for communicating with an ultrasound apparatus that acquires the selected ultrasound image; and a control unit for controlling the display unit, the user input unit, and the communication unit.

The ultrasound image list may include a real-time ultrasound image transmitted from the at least one ultrasound apparatus and an ultrasound image stored in advance.

The control unit may indicate an identification mark on the real-time ultrasound image to distinguish the real-time ultrasound image from the ultrasound image stored in advance.

The display unit may display at least one of analysis information of a sonographer about the selected ultrasound image, measurement information with respect to the selected ultrasound image, and examinee information, according to a sharing level of the medical expert device.

The display unit may display a pointer of the ultrasound apparatus and a pointer of the medical expert device on the selected ultrasound image.

The display unit may provide a chatting window for communicating with the ultrasound apparatus on a third region of the screen, the user input unit may receive an input about the selected ultrasound image through the chatting window, and the communication unit may transmit the received input to the ultrasound apparatus.

The communication unit may receive a request for confirmation about the selected ultrasound image from the ultrasound apparatus, and transmit the confirmation information to the ultrasound apparatus.

The display unit may display a graphical user interface (GUI) for receiving a confirmation input of the selected ultrasound image, and receive a confirmation input about the selected ultrasound image through the GUI.

The communication unit may transmit control information for controlling the ultrasound apparatus to the ultrasound apparatus.

The user input unit may receive a description by a medical expert about the selected ultrasound image, and the communication unit may transmit the description by the medical expert to the examinee device.

According to another aspect of an example, there is provided a method of sharing information in a medical imaging apparatus, the method including: acquiring a medical image of an object; selecting an external device for sharing information about the medical image; identifying a sharing level of the external device that is selected; and transmitting the information about the medical image to the external device based on the sharing level.

The sharing level may include information representing an authority of a user of the external device for checking the information about the medical image.

The external device may include a first device and a second device, and the transmitting of the information may include: transmitting first medical image information corresponding to a first sharing level of the first device to the first device; and transmitting second medical image information corresponding to a second sharing level of the second device to the second device.

The above and other features and advantages of the present invention will become more apparent by describing in detail exemplary embodiments thereof with reference to the attached drawings in which:
FIG. 1 is a block diagram of an information sharing system according to an example of the present disclosure;
FIG. 2 is a block diagram of an information sharing system according to another example of the present disclosure;
FIG. 3 is a flowchart illustrating an information sharing method of an ultrasound apparatus, according to an example of the present disclosure;
FIG. 4 is a flowchart illustrating a method of generating ultrasound information, according to an example of the present disclosure;
FIG. 5 is a flowchart illustrating a method of generating first ultrasound information corresponding to a sharing level of a first device, by an ultrasound apparatus, according to an example of the present disclosure;
FIG. 6 is a diagram showing a graphical user interface (GUI) for adding an annotation, according to an example of the present disclosure;
FIG. 7 is a diagram showing a GUI for marking a region of interest according to an example of the present disclosure;
FIG. 8 is a diagram showing a GUI for adding an annotation and a color, according to an example of the present disclosure;
FIG. 9 is a diagram showing a screen of a first device according to an example of the present disclosure;
FIG. 10 is a flowchart illustrating a communication method between the ultrasound apparatus according to the example of the present disclosure and a second device;
FIG. 11 is a diagram showing a GUI for confirming an ultrasound image, according to an example of the present disclosure;
FIG. 12 is a diagram showing a screen providing a chatting window in an ultrasound apparatus, according to an example of the present disclosure;
FIG. 13 is a diagram showing a screen displaying a message in an ultrasound apparatus, according to an example of the present disclosure;
FIG. 14 is a flowchart illustrating a communication method of a medical expert device, according to an example of the present disclosure;
FIG. 15 is a diagram showing a screen providing a list of ultrasound images in a medical expert device, according to an example of the present disclosure;
FIG. 16 is a diagram showing a screen displaying a chatting window and a pointer in a medical expert device, according to an example of the present disclosure;
FIG. 17 is a diagram showing an example of a GUI for remotely controlling the ultrasound apparatus by the medical expert device, according to the example of the present disclosure;
FIGS. 18A and 18B are diagrams showing an example of a GUI for adding a body marker to the ultrasound image remotely by the medical expert device, according to the example of the present disclosure;
FIG. 19 is a flowchart illustrating a communication method between the medical expert device and the examinee device, according to the example of the present disclosure;
FIGS. 20A through 20C are diagrams showing screens of the ultrasound apparatus, the first device, and the second device according to the example of the present disclosure;
FIG. 21 is a block diagram showing an ultrasound apparatus according to an example of the present disclosure;
FIG. 22 is a block diagram showing an ultrasound apparatus according to another example of the present disclosure;
FIG. 23 is a block diagram showing a medical expert device according to an example of the present disclosure;
FIG. 24 is a flowchart illustrating an information sharing method of a medical imaging apparatus according to an example of the present disclosure;
FIG. 25 is a diagram showing an X-ray image obtained by an X-ray photographing apparatus according to an example of the present disclosure;
FIG. 26 is a diagram showing a Bone suppression image, in which a region of interest is marked, according to an example of the present disclosure;
FIG. 27 is a diagram showing an X-ray image including an annotation according to an example of the present disclosure.

General and widely-used terms have been employed herein, in consideration of functions provided in the present disclosure, and may vary according to an intention of one of ordinary skill in the art, a precedent, or emergence of new technologies. Additionally, in some cases, an applicant may arbitrarily select specific terms, in which case, the applicant will provide the meaning of the terms in the description of the present disclosure. Accordingly, It will be understood that the terms used herein should be interpreted as having a meaning that is consistent with their meaning in the context of the relevant art and will not be interpreted in an idealized or overly formal sense unless expressly so defined herein.

It will be further understood that the terms "comprises," "comprising," "includes," and/or "including," when used herein, specify the presence of components, but do not preclude the presence or addition of one or more other components, unless otherwise specified. Additionally, terms used herein, such as 'unit' or 'module', mean entities for processing at least one function or operation. These entities may be implemented by hardware, software, or a combination of hardware and software. Expressions such as "at least one of," when preceding a list of elements, modify the entire list of elements and do not modify the individual elements of the list.

In the present specification, 'ultrasound image' is an image about an object acquired by using ultrasound signals. The object may denote a human body part. For example, the object may include an organ such as a liver, heart, brain, breast, and abdomen, or a fetus, or nuchal translucency (NT).

The ultrasound image may be realized in various modes. For example, the ultrasound image may be at least one of a brightness mode (B mode) image representing a magnitude of an ultrasound echo signal reflected by an object as brightness, a color mode (C mode) image representing a velocity of a moving object as a color by using a Doppler effect, a Doppler mode (D mode) image representing an object of a moving object as a spectrum by using a Doppler effect, a motion mode (M mode) image representing movement of an object at a constant location according to time, and an elastic mode image representing a difference between reactions when a compression is applied and not applied to an object as an image; however, the present disclosure is not limited thereto. Also, according to examples of the present disclosure; the ultrasound image may be a two-dimensional (2D) image, a three-dimensional (3D) image, or a four-dimensional (4D) image.

The present disclosure will now be described more fully with reference to the accompanying drawings, in which examples of the disclosure are shown. The disclosure may, however, be embodied in many different forms and should not be construed as being limited to the examples set forth herein. In the description of the present disclosure, certain detailed explanations of the related art will be omitted when it is deemed that they may unnecessarily obscure the essence of the disclosure. Like numbers refer to like elements throughout the description of the figures. As used herein, the term "and/or" includes any and all combinations of one or more of the associated listed items. Expressions such as "at least one of," when preceding a list of elements, modify the entire list of elements and do not modify the individual elements of the list.

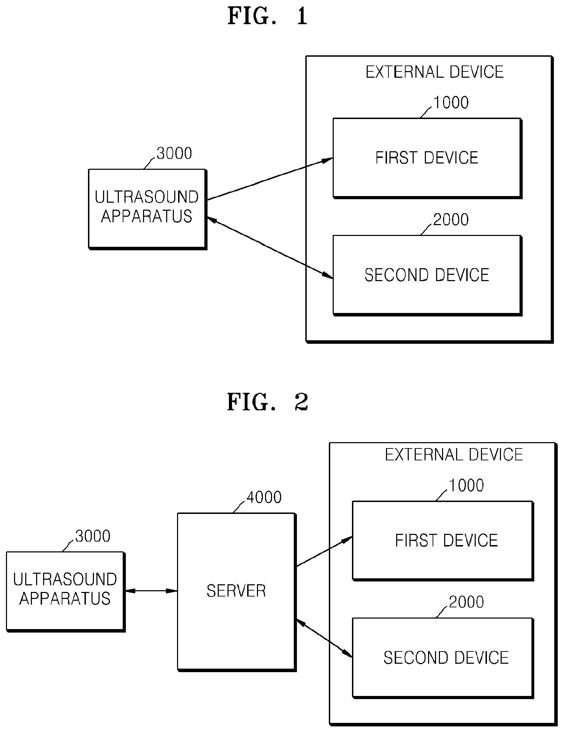

FIG. 1 is a block diagram of an information sharing system according to an example of the present disclosure.

The information sharing system according to the present example may include an ultrasound apparatus 3000 and an external device (for example, at least one of a first device 1000 and a second device 2000).

According to the present example, the first device 1000 may be a display apparatus for providing an ultrasound image. For example, the first device 1000 may be a device for an examiner, which displays an ultrasound image for the examinee to view (hereinafter, referred to as 'examinee device').

The first device 1000 may receive ultrasound information about an ultrasound image from the ultrasound apparatus 3000. The first device 1000 may receive the ultrasound information directly from the ultrasound apparatus 3000, or via a server.

The ultrasound device received by the first device 1000 may be ultrasound information corresponding to a sharing level of the first device 1000. For example, the ultrasound information corresponding to the sharing level of the first device 1000 may include ultrasound image information, caption (or annotation) information for illustrating all or part of the ultrasound image, and identification mark information for identifying a region of interest in the ultrasound image; however, the present disclosure is not limited thereto.

The sharing level of the first device 1000 may be set by an information sharing system or a user, in advance.

The second device 2000 may be a device of a medical expert, which may perform a data communication with the ultrasound apparatus 3000 (hereinafter, referred to as 'medical expert device'). In the present specification, a medical expert may denote an expert who can analyze or confirm an ultrasound image transmitted from the ultrasound apparatus 3000 by using the second device 2000. For example, the medical expert may include a doctor, a sonographer, a medical technologist, a nurse, or a radiological technologist; however, the present disclosure is not limited thereto.

Also, the medical expert according to the present example may be different from a sonographer who acquires an ultrasound image by using the ultrasound apparatus 3000. For example, if a first sonographer acquires an ultrasound image of an object by using the ultrasound apparatus 3000, a second sonographer or a doctor may identify the ultrasound image acquired by the first sonographer or analysis information generated by the first sonographer by using the second device 2000.

The second device 2000 according to the present example may receive ultrasound information about the ultrasound image from the ultrasound apparatus 3000, and may display the ultrasound information on a screen thereof. Here, the second device 2000 may receive the ultrasound information corresponding to a sharing level of the second device 2000.

According to the present example, the second device 2000 and the first device 1000 may have different sharing levels from each other. Therefore, the ultrasound information corresponding to the sharing level of the second device 2000 may be different from ultrasound information corresponding to the sharing level of the first device 1000.

For example, the ultrasound information corresponding to the sharing level of the second device 2000 may include analysis information generated by the sonographer, information about an examinee, and measurement information; however, the present disclosure is not limited thereto.

In addition, the second device 2000 according to the present example may be a device having an authority to control the ultrasound apparatus 3000 remotely. That is, the second device 2000 may transmit control information for controlling the ultrasound apparatus 3000 based on an input of the medical expert (for example, a doctor).

For example, the second device 2000 may transmit to the ultrasound apparatus 300 a control command for selecting and displaying another ultrasound image, a control command for expanding or reducing the ultrasound image, a control command for storing the ultrasound image, a control command for performing a 3D rendering of the ultrasound image, a control command for adding an annotation or a body marker, a control command for measuring a region of interest, and a control command for correcting analysis information about the ultrasound image.

The ultrasound apparatus 3000 of the present example denotes a device that acquires ultrasound image data of an object by using ultrasound waves and shares ultrasound information about the ultrasound image with an external device.

The ultrasound apparatus 3000 of the present example may be realized as various types. For example, the ultrasound apparatus 3000 may be configured as a mobile terminal, as well as a fixed terminal. Examples of the mobile terminal may include a laptop computer, a personal digital assistant (PDA), a tablet personal computer (PC), and a smartphone.

The ultrasound apparatus 3000 of the present example may transmit the ultrasound information to a receiving external device (for example, the first device 1000 or the second device 2000) according to the sharing level of the receiving device. That is, the ultrasound apparatus 3000 may determine a kind and/or amount of pieces of information to be transmitted to the receiving device, from among the information relating to the ultrasound image, according to the sharing level of the receiving device.

For example, the ultrasound apparatus 3000 may transmit different ultrasound information respectively to the examinee display device and the medical expert display device. When performing the ultrasound diagnosis, there is no need to expose a lot of information to the examinee, and thus, an amount of ultrasound information transferred to the examinee display device may be less than that of ultrasound information transferred to the medical expert display device. Also, an annotation or a color may be added to the ultrasound information that is transmitted to the examinee display apparatus.

The ultrasound apparatus 3000 of the present example may transmit the ultrasound information in various ways. For example, the ultrasound apparatus 3000 may encode ultrasound information about an ultrasound image by using a mirroring technology using a miracast, and may transmit the encoded ultrasound information to the first device 1000 or the second device 2000 via Wi-Fi Direct (WFD). An encoding algorithm according to the present example may include MPEG-2, MPEG-4, H.264, or advanced video coding (AVC); however, examples of the present disclosure are **not** limited thereto.

In addition, the ultrasound apparatus 3000 may transmit the ultrasound information to the first device 1000 or the second device 2000 via wireless fidelity (Wi-Fi), Bluetooth, ultra wideband (UWB), or IEEE 1394 communication, but examples of the present disclosure are not limited thereto.

The ultrasound apparatus 3000 according to the present example may include a touch screen. The touch screen may be configured to detect a touch input location, a touched area, and a touch input pressure. Also, the touch screen may detect a proximity touch, as well as a real-touch input.

In the present specification, the real-touch input denotes a case where the screen is actually touched by a touch tool (for example, a finger, an electronic pen, etc.), and the proximity touch input denotes a case where a touch tool approaches the screen to within a predetermined distance without actually touching the screen.

The ultrasound apparatus 3000 may sense a touch gesture of a user on an ultrasound image via the touch screen.

Touch gestures of a user may include a tap, a touch and hold, a double tap, a drag, panning, a flick, a drag and drop, a swipe, a pinch, etc.

"Tap" refers to an operation in which the user touches the screen by using a finger or an electronic pen and then lifts the finger or the electronic pen from the screen without moving it on the screen.

"Touch & hold" is an operation in which the user touches the screen by using a finger or an electronic pen and maintains the touch input for a critical length of time (for example, two seconds) or longer. That is, a time difference between a touch-in time and a touch-out time is equal to or greater than the critical length of time (for example, two seconds). In order for the user to recognize whether the touch input is the tap operation or the touch & hold operation, a visual, an audible, or a tactile feedback signal is transmitted when the touch input is maintained for the critical length of time or longer. The critical length of time may vary depending on examples.

"Double tap" is an operation in which the user touches the screen twice by using the finger or the electronic pen.

"Drag" is an operation in which the user touches the screen by using a finger or an electronic pen and then moves the finger or the electronic pen to another position on the screen while continuously touching the screen. An object is moved or a panning operation that will be described later is performed by the drag operation.

"Panning" is an operation in which the user performs the drag operation without selecting an object. Since the user does not select a certain object in the panning operation, a page itself moves in the screen or a group of objects moves in the page, without moving the certain object in the page.

"Flick" is an operation in which the user drags a finger or an electronic pen at a critical speed (for example, 100 pixel/s) or faster. The drag operation (or panning operation) and the flick operation may be distinguished from each other based on whether the velocity of the finger or the electronic pen is the critical speed (for example, 100 pixel/s) or greater.

"Drag & drop" is an operation in which the user drags and drops an object at a predetermined location on the screen by using the finger or the electronic pen.

"Pinch" is an operation in which the user touches the screen by using two fingers and then moves the fingers to different directions from each other. The pinch operation is a gesture for expanding (pinch open) or reducing (pinch close) the object or the page, and an expansion value or a reduction value may be determined by a distance between the two fingers.

"Swipe" is an operation in which the user moves the finger or the electronic pen a predetermined distance in a horizontal or a vertical direction in a state of touching the object on the screen. Movement in a diagonal direction may not be considered as a swipe event.

The ultrasound apparatus 3000 of the present example may provide some or all of buttons included in a control panel of a general ultrasound apparatus through the touch screen as a graphical user interface (GUI).

FIG. 2 is a block diagram of an information sharing system according to another example of the present disclosure.

The information sharing system according to the present example may further include a server 4000, in addition to the first device 1000, the second device 2000, and the ultrasound apparatus 3000. Descriptions of the first device 1000, the second device 2000, and the ultrasound apparatus 3000 will not be repeated here, and only the server 4000 will be described below.

The server 4000 may be a server for managing information related to ultrasound images. The server 4000 according to the present example may include a server of a medical institution (for example, a hospital).

The server 4000 of the present example may store information about examinees, and may store information relating to ultrasound images captured by the ultrasound apparatus 3000. Also, the server 4000 may store information about sharing levels of devices that may share information with the ultrasound apparatus 3000. For example, information such as identification information of a device (for example, a device ID, a Mac address, and an equipment name, etc.), the sharing level, kinds of information that may be shared, and an amount of data that may be shared may be stored in the server 4000 in a table format.

The server 4000 of the present example may transmit information about real-time ultrasound images received from the ultrasound apparatus 3000 to the first device 1000 or the second device 2000. The real-time ultrasound image may refer to an ultrasound image that is captured by the ultrasound apparatus 3000 within a predetermined time period (for example, within five minutes) from the present or at a time of receiving the ultrasound image.

The server 4000 according to the present example may receive a request to transmit a certain ultrasound image that is stored in advance from the second device 2000, and then, may transmit the requested ultrasound image to the second device 2000.

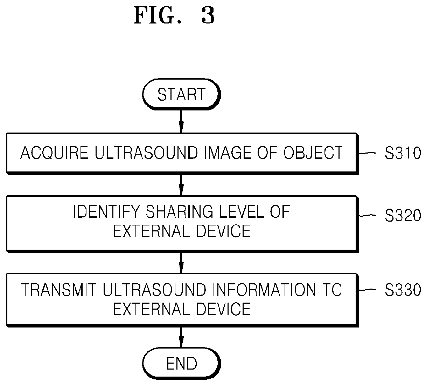

Hereinafter, a method of sharing ultrasound information about ultrasound images by the ultrasound apparatus 3000 with a plurality of other devices according to a sharing level will be described in more detail with reference to FIG. 3.

FIG. 3 is a flowchart illustrating an information sharing method of an ultrasound apparatus 3000, according to an example of the present disclosure.

In operation S310, the ultrasound apparatus 3000 may acquire an ultrasound image about an object. The ultrasound apparatus 3000 of the present example may directly generate the ultrasound image, or may receive the ultrasound image from outside.

For example, the ultrasound apparatus 3000 transmits an ultrasound signal to the object and receives an ultrasound response signal from the object to generate the ultrasound image. Otherwise, the ultrasound apparatus 3000 may receive an ultrasound image from an external server or an external device.

In operation S320, the ultrasound apparatus 3000 may identify a preset sharing level of an external device for sharing the ultrasound image. For example, the ultrasound apparatus 3000 may identify information about a first sharing level corresponding to identification information of the first device 1000 and/or a second sharing level corresponding to identification information of the second device 2000.

According to the present example, the ultrasound apparatus 3000 may receive information about the first sharing level and/or information about the second sharing level from the server 4000. In addition, according to another example of the present disclosure, the ultrasound apparatus 3000 may read the information about the first sharing level and/or the information about the second sharing level from a memory.

According to the present example, the first sharing level of the first device 1000 and the second sharing level of the second device 2000 may be different from each other. For example, if the first device 1000 is the examinee device and the second device 2000 is the medical expert device, the sharing level of the first device 1000, that is, the examinee device, may be lower than the sharing level of the second device 2000, that is, the medical expert device. The lower sharing level denotes that a number of kinds and/or amounts of the sharable information are relatively small.

In operation S330, the ultrasound apparatus 3000 may transmit ultrasound information to the external device according to the preset sharing level. That is, the ultrasound apparatus 3000 may transmit first ultrasound information corresponding to the first sharing level to the first device 1000, or may transmit second ultrasound information corresponding to the second sharing level to the second device 2000.

For example, if the first device 1000 is the examinee device and the second device 2000 is the medical expert device, the ultrasound apparatus 3000 shares the ultrasound image only with the first device 1000, and may share additional information in addition to the ultrasound information with the second device 2000. The additional information may include analysis information of a sonographer, measurement information, and examinee information; however, the example of the present disclosure is **not limited thereto.**

According to the present example, the external device (for example, the first device 1000 and/or the second device 2000) may display the ultrasound information transmitted from the ultrasound apparatus 3000 in real-time. The displaying of the ultrasound information in real-time may denote displaying of the ultrasound information on the screen within a predetermined time period (for example, one minute) from a point of time when the ultrasound information is received. For example, the first device 1000 may display first ultrasound information, and the second device 2000 may display second ultrasound information in real-time.

In addition, according to the present example, the ultrasound apparatus 3000 may encode the first ultrasound information by using an encoding code that is negotiated in advance or an encoding code that is set in advance for securing the first ultrasound information, and may transmit the encoded first ultrasound information to the first device 1000. Also, the ultrasound apparatus 3000 encodes the second ultrasound information by using an encoding code that is negotiated in advance or an encoding code that is set in advance for securing the second ultrasound information, and may transmit the encoded second ultrasound information to the second device 2000.

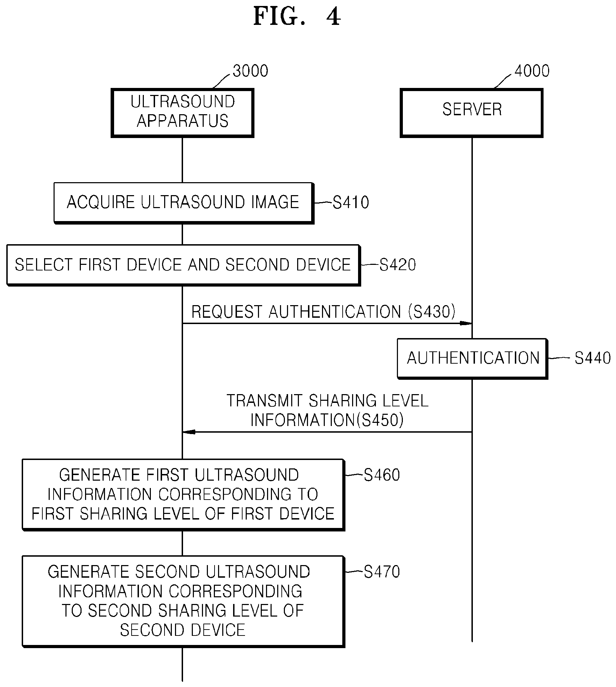

Hereinafter, the method of sharing the ultrasound information by the ultrasound apparatus 3000 according to the sharing level will be described in more detail with reference to FIGS. 4 through 13 . For convenience of description, it is assumed that the first device 1000 is an examinee device and the second device 2000 is a medical expert device.

FIG. 4 is a flowchart illustrating a method of generating ultrasound information, according to an example of the present disclosure.

In operation S410, the ultrasound apparatus 3000 may acquire an ultrasound image of an object. For example, the ultrasound apparatus 3000 may acquire a B mode image, a C mode image, a D mode image, an M mode image, an elastic mode image, a 2D ultrasound image, a 3D ultrasound image, and a 4D ultrasound image of the object; however, the example is not limited thereto. Since operation S410 corresponds to operation S310 shown in FIG. 3 , detailed descriptions thereof will not be provided here.

In operation S420, the ultrasound apparatus 3000 may select the first device 1000 and the second device 2000 with which the information will be shared. Here, according to the present example, the ultrasound apparatus 3000 may select the first device 1000 and the second device 2000 connected to the ultrasound apparatus 3000 based on system settings.

According to another example of the present disclosure, the ultrasound apparatus 3000 may select the first device 1000 and the second device 2000 based on a user input. For example, the ultrasound apparatus 3000 may provide a device list including identification information of the devices that may communicate with the ultrasound apparatus 3000 on a screen thereof. In addition, the ultrasound apparatus 3000 may receive the user input for selecting the first device 1000 and the second device 2000 in the device list. The user of the ultrasound apparatus 3000 of the present example may be a sonographer, an emergency medical technician, or an ordinary person; however, the example of the present disclosure is not limited thereto. Hereinafter, the sonographer will be assumed as the user of the ultrasound apparatus 3000.

In operation S430, the ultrasound apparatus 3000 may request authentication regarding the first device 1000 and the second device 2000 to the server 4000. In operation S440, the server 4000 may perform authentication operations regarding the first device 1000 and the second device 2000. For example, the server 4000 may verify whether each of the first device 1000 and the second device 2000 is a device that may share the information with the ultrasound apparatus 3000. Through the authentication, exposure of the ultrasound information relating to personal medical diagnosis to unspecified public may be prevented.

In operation S450, when the authentication of the first device 1000 and the second device 2000 has succeeded, the server 4000 may transmit the information about the sharing information of the first device 1000 and the second device 2000 to the ultrasound apparatus 3000. That is, the ultrasound apparatus 3000 may receive information about the first sharing level of the first device 1000 and the information about the second sharing level of the second device 2000 from the server 4000.

In operation S460, the ultrasound apparatus 3000 may generate the first ultrasound information corresponding to the first sharing level of the first device 1000. For example, when the first device 1000 is the examinee device, the ultrasound apparatus 3000 may generate the first ultrasound information including an ultrasound image, annotation for describing the ultrasound image, an identification mark on a region of interest, and measurement information according to the sharing level of the examinee device.

The annotation of the present example may include an annotation representing the object (for example, a head of an embryo, liver, heart, carotid, etc.), and an annotation describing a kind of the ultrasound image (for example, a Doppler image, an M mode image, an elastic mode image, etc.); however, the examples of the present disclosure are not limited thereto.

The identification mark on the region of interest according to the present example may include adding of a color to the region of interest (for example, adding of skin color to the embryo image), adding of a boundary to the region of interest, and adding of a pattern to the region of interest; however, the examples of the present disclosure are **not** limited thereto.

The measurement information according to the present example may include information obtained from measuring the region of interest. For example, the measurement information may include a head girth and a neck girth of an embryo, a head size, a tumor size, etc.; however, the examples of the present disclosure are not limited thereto.

In operation S470, the ultrasound apparatus 3000 may generate the second ultrasound information corresponding to the second sharing level of the second device 2000. For example, when the second device 2000 is a medical expert device, the ultrasound apparatus 3000 generates the second ultrasound information including an ultrasound image, measurement information about the ultrasound image, analysis information of the sonographer about the ultrasound image, and examinee information according to the sharing level of the medical expert device.

The measurement information provided to the medical expert device may include more detailed information than that provided to the examinee device. For example, the measurement information provided to the medical expert device may include a maximum speed in a sample volume, an inclination in an M mode image, etc., in addition to the head girth and a neck girth of the embryo, head size, and tumor size, etc.

The analysis information of the sonographer according to the present example may include a result report generated by the sonographer after performing an ultrasound wave examination. The examinee information of the present example may include a medical history of the examinee, heath state information of the examinee, body features of the examinee, and image history of the examinee; however, the examples of the present disclosure are not limited thereto.

Therefore, according to the present example, the ultrasound apparatus 3000 may transmit different pieces of the ultrasound information about one ultrasound image acquired from the object respectively to the examinee device and the medical expert device according to the sharing level.

According to examples of the present disclosure, an order of performing operations S410 to S470 may be changed, and some of the operations may be omitted.

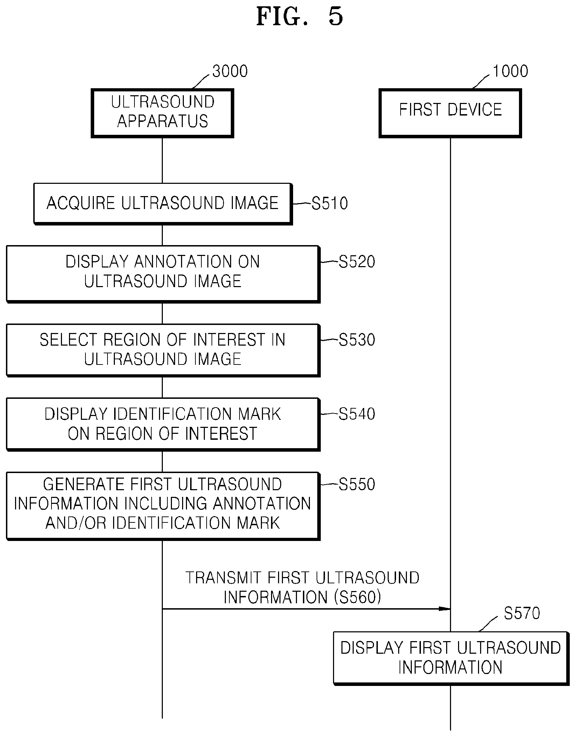

Hereinafter, the first ultrasound information shared by the ultrasound apparatus 3000 with the first device 1000 will be described in detail with reference to FIGS. 5 through 9 . Here, as described above, the examinee device will be described as an example of the first device 1000.

FIG. 5 is a flowchart illustrating a method of generating the first ultrasound information by the ultrasound apparatus 3000 to correspond to the sharing level of the first device 1000, according to an example of the present disclosure.

In operation S510, the ultrasound apparatus 3000 may acquire an ultrasound image of an object. Since operation S510 corresponds to operation S310 shown in FIG. 3 , detailed descriptions thereof will be omitted here.

In operation S520, the ultrasound apparatus 3000 may add at least one annotation to the ultrasound image. That is, the ultrasound apparatus 3000 may add at least one annotation relating to all or some of the ultrasound image to the ultrasound image.

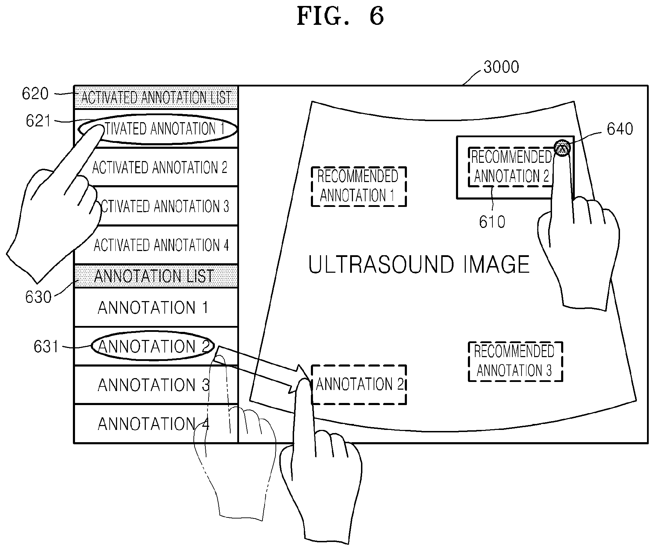

According to the present example, the ultrasound apparatus 3000 may analyze the ultrasound image to recommend at least one annotation. Also, the ultrasound apparatus 3000 may add at least one annotation to the ultrasound image based on a selection of the user (for example, the sonographer). For example, the ultrasound apparatus 3000 may display an annotation list that is set in advance. The ultrasound apparatus 3000 receives the user's selection of at least one annotation in the annotation list, and then, may display the selected annotation on the ultrasound image. The adding of the annotation by the ultrasound apparatus 3000 will be described later with reference to FIG. 6.

In operation S530, the ultrasound apparatus 3000 may select a region of interest in the ultrasound image. According to the present example, the ultrasound apparatus 3000 may select the region of interest automatically by analyzing the ultrasound image, or based on the user input.

For example, the ultrasound apparatus 3000 may detect an edge from the ultrasound image and recommend the region of interest automatically. Otherwise, the ultrasound apparatus 3000 may display a graphical user interface (GUI) allowing the user to set the region of interest directly on the screen.

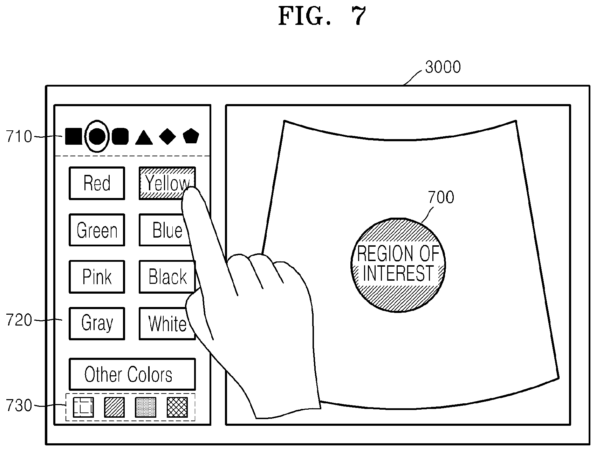

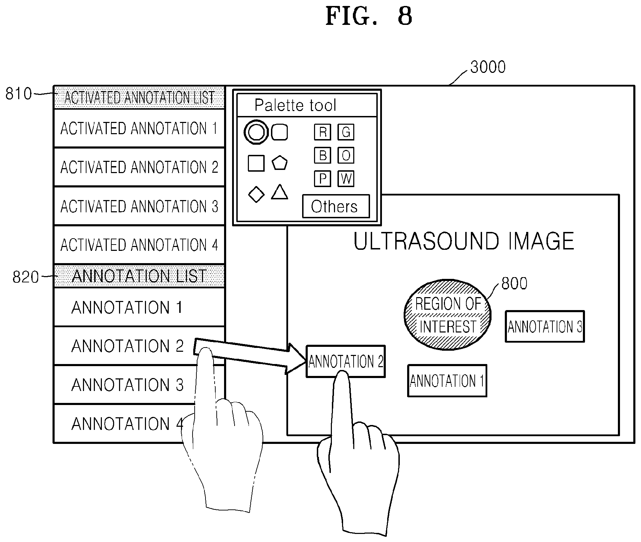

In operation S540, the ultrasound apparatus 3000 may display an identification mark on the region of interest. For example, the ultrasound apparatus 3000 may add a color or a pattern in the region of interest, or may change a thickness of a boundary on the region of interest. This will be described in more detail with reference to FIGS. 7 and 8.

In operation S550, the ultrasound apparatus 3000 may generate first ultrasound information including at least one of the annotation and the identification mark on the region of interest. For example, the ultrasound apparatus 3000 may generate the first ultrasound information including an ultrasound image, to which an annotation for the examinee's comprehension is added. Also, the ultrasound apparatus 3000 may generate the first ultrasound information in which the color or the pattern is added to the region of interest, or the first ultrasound information including the annotation and the region of interest on which the identification mark is displayed.

In operation S560, the ultrasound apparatus 3000 may transmit the first ultrasound information to the first device 1000. According to the present example, the ultrasound apparatus 3000 may transmit the first ultrasound information to the first device 1000 via short distance communication. The short distance communication may be Wireless Local Area Network (WLAN) (e.g., Wireless Fidelity (Wi-Fi)), Bluetooth, Bluetooth Low Energy (BLE), UWB, ZigBee, and WFD; however, the examples are not limited thereto.

In operation S570, the first device 1000 receives the first ultrasound information from the ultrasound apparatus 3000, and may display the received first ultrasound information on a screen thereof. The examinee may identify the first ultrasound information relating to the ultrasound image acquired by the ultrasound apparatus 3000 via the first device 1000.

According to the examples, an order of performing operations S510 to S570 may be changed, and some operations may be omitted.

Hereinafter, the GUI for generating the first ultrasound information will be described in more detail with reference to FIGS. 6 through 8.

FIG. 6 is a diagram showing a GUI for adding an annotation, according to an example of the present disclosure.

As shown in FIG. 6 , the ultrasound apparatus 3000 analyses the information about the ultrasound image to recommend annotations for describing a part or all of the ultrasound image (for example, a recommended annotation 1, a recommended annotation 2, and a recommended annotation 3). For example, the ultrasound apparatus 3000 may analyze the information about the ultrasound image, and may display a recommended annotation for describing the object or a recommended annotation for representing a location of an embryo head at corresponding locations. A method of analyzing the ultrasound image by the ultrasound apparatus 3000 is well known in the art, and thus, detailed descriptions thereof will not be provided here.

According to the present example, the ultrasound apparatus 3000 may move, delete, or edit a recommended annotation 610 based on a user input. For example, when a touch input for touching the recommended annotation 610 for a predetermined time or longer is received, the ultrasound apparatus 3000 may display a delete icon 640 for deleting the recommended annotation 610. In this case, the user may select the delete icon 640 for deleting the recommended annotation 610.

Also, when the touch input for touching the recommended annotation 610 is maintained for a predetermined length of time or longer, the ultrasound apparatus 3000 may enable the recommended annotation 610 to move. In this case, the user may drag the recommended annotation 610 to change the display location of the recommended annotation 610.

According to the present example, the ultrasound apparatus 3000 may provide an annotation list 630 that is set in advance on a predetermined region of the screen for the user's selection. Here, the annotation list 630 of the present example may be displayed on a region that is different from the region where the ultrasound image is displayed.

According to the present example, the at least one annotation included in the annotation list 630 may be arranged based on usage times thereof. For example, the annotations in the annotation list 630 may be arranged in an order from most frequently used by the user.

Also, the annotation list 630 of the present example may include annotations relating to the current ultrasound mode and set in advance, or annotations relating to the object and set in advance. For example, if the object is an embryo, annotations relating to the embryo may be included in the annotation list 630. The annotation list 630 of the present example may include annotations relating to the identification information of the sonographer or identification information of the examinee.

The ultrasound apparatus 3000 of the present example may receive a selection of the user (for example, the sonographer) on at least one annotation from the annotation list 630 and may display the selected annotation on the ultrasound image.

For example, the ultrasound apparatus 3000 may sense a drag and drop input which involves dragging a second annotation 631 from the annotation list 630 to the region where the ultrasound image is displayed and dropping the second annotation 631 onto a certain point. In this case, the ultrasound apparatus 3000 may display the second annotation 631 at the certain point where the drop input is sensed, based on the drag and drop input.

According to the present example, the ultrasound apparatus 3000 may provide an activated annotation list 620 including identification information of the annotations displayed on the ultrasound image on a predetermined region of the screen. Here, the activate annotation may denote an annotation that is currently displayed on the ultrasound image. For example, the recommended annotation 1, the recommended annotation 2, the recommended annotation 3, and the second annotation displayed on the ultrasound image may be activated annotations. The user may identify the annotations that are added to the current ultrasound image via the activated annotation list 620.

According to the present example, the ultrasound apparatus 3000 may receive a user input for selecting at least one activated annotation from the activated annotation list 620. In this case, the ultrasound apparatus 3000 may activate an editing mode of the selected activated annotation.

For example, the user may touch an activated annotation 1 621 from the activated annotation list 620 for a predetermined time, tap the activated annotation 1 621, or double-tap the activated annotation 1 621. Then, the ultrasound apparatus 3000 may activate an edition mode of the activated annotation 1 621, and may correct the activated annotation 1 621 displayed on the ultrasound image.

FIG. 7 is a diagram showing a GUI for displaying an identification mark on a region of interest, according to an example of the present disclosure.

As shown in FIG. 7 , the ultrasound apparatus 3000 of the present example may provide a template list 710 for selecting a region of interest 700. The template may be a figure that is set in advance and used to select the region of interest 700. The template list 710 according to the present example may include a circle, a square, a pentagon, etc.

For example, if the user selects a circle from the template list 710, the user may select the region of interest 700 by changing a location and a size of the circle on the ultrasound image.

According to another example of the present disclosure, the user may select the region of interest 700 by directly drawing a line on the ultrasound image by using a touch tool (for example, a finger, and an electronic pen), a mouse, or a trackball.

The ultrasound apparatus 3000 of the present example may provide a color list 720 for adding a color to the region of interest 700. Also, the ultrasound apparatus 3000 according to example of the present disclosure may provide a pattern list 730 so that the user may add a pattern to the region of interest 700. Here, the user (for example, the sonographer) may add the color or the pattern to the region of interest 700 so as to help the examinee's comprehension.

FIG. 8 is a diagram showing a GUI for adding an annotation and a color, according to an example of the present disclosure.

As shown in FIG. 8 , the ultrasound apparatus 3000 may provide an activated annotation list 810, an annotation list 820, and a palette tool 830. In this case, the user (for example, the sonographer) may add an annotation for describing the ultrasound image. Also, the user (for example, the sonographer) may select the region of interest 800 in the ultrasound image, and may indicate an identification mark on the region of interest 800 by using the palette tool 830.

The ultrasound apparatus 3000 of the present example may transmit first ultrasound information corresponding to the sharing level of the first device 1000 to the first device 1000. If the first device 1000 is the examinee device, the sharing level information of the first device 1000 may be set in advance to share the annotation and the identification mark on the ultrasound image, as well as the ultrasound image, with the first device 1000. Therefore, the ultrasound apparatus 3000 of the present example may transmit the first ultrasound information including the annotation and the identification mark on the region of interest for helping the examinee's comprehension of the ultrasound image to the first device 1000.

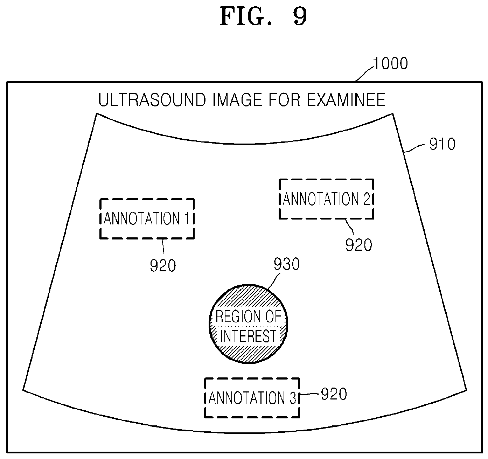

FIG. 9 is a diagram showing a screen of the first device 1000, according to an example of the present disclosure.

As shown in FIG. 9 , the first device 1000 of the present example may display the first ultrasound information transmitted from the ultrasound apparatus 3000 on the screen thereof. Here, the first ultrasound information displayed on the screen may include an ultrasound image 910, an annotation 920 for describing the ultrasound image 910, and a region of interest 930 indicated with an identification mark.

For example, if the ultrasound image 910 is an image of an embryo, the first device 1000 may display skin color on the image of the embryo so that the user may identify the embryo easily. Also, the first device 1000 may display information about a head girth of the embryo, a location of the head, etc. as an annotation so that the examinee may easily understand the image.

Therefore, the examinee may understand the ultrasound image captured by the ultrasound apparatus 3000 through the first ultrasound information displayed on the first device 1000.

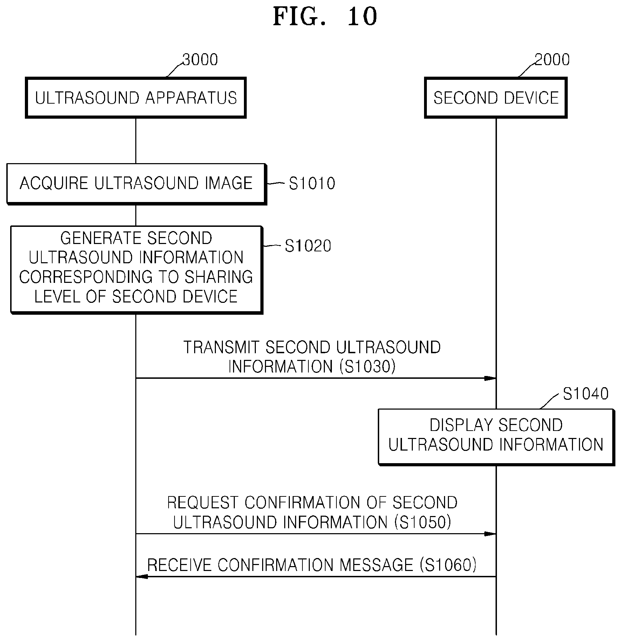

Hereinafter, processes of sharing the second ultrasound information between the ultrasound apparatus 3000 and the second device 2000 will be described with reference to FIGS. 10 through 13 .

FIG. 10 is a flowchart illustrating a communication method between the ultrasound apparatus 3000 and the second device 1000, according to an example of the present disclosure.

In operation S1010, the ultrasound apparatus 3000 may acquire an ultrasound image about an object. Since operation S1010 corresponds to operation S310 shown in FIG. 3 , detailed descriptions thereof will not be provided here.

In operation S1020, the ultrasound apparatus 3000 may generate second ultrasound information corresponding to the sharing level of the second device 2000. For example, if the second device 2000 is a medical expert device, the ultrasound apparatus 3000 may generate the second ultrasound information including an ultrasound image, measurement information of the ultrasound image, analysis information of the sonographer about the ultrasound image, and the examinee information. Since operation S1020 corresponds to operation S470 of FIG. 4 , detailed descriptions thereof will not be provided.

In operation S103, the ultrasound apparatus 3000 may transmit the second ultrasound information to the second device 2000. According to the present example, the ultrasound apparatus 3000 may transmit the second ultrasound information to the second device 2000 via short distance communication. Examples of the short distance communication may include Wi-Fi, near field communication (NFC), Bluetooth, BLE, Zigbee, WFD, and UWB; however, the examples of the present disclosure are **not limited thereto.**

In operation S1040, the second device 2000 receives the second ultrasound information transmitted from the ultrasound apparatus 3000, and may display the second ultrasound information on a screen thereof. The medical expert (for example, a doctor) may identify the ultrasound image acquired by the ultrasound apparatus 3000 via the second device 2000. Also, the medical expert (for example, the doctor) may identify the analysis information of the sonographer, the measurement information of the ultrasound image, and the examinee information included in the second ultrasound information so as to diagnose a disease of the examinee exactly.

In operation S1050, the ultrasound apparatus 3000 may transmit a request for confirmation of the second ultrasound information to the second device 2000. For example, the ultrasound apparatus 3000 may request a confirmation of the medical expert (for example, the doctor) for the ultrasound image or the analysis information of the sonographer included in the second ultrasound information.

In operation S1060, the second device 2000 may transmit a message for confirming the second ultrasound information to the ultrasound apparatus 3000 in response to the request for confirmation. For example, the second device 2000 may receive confirmation information of the ultrasound image from the medical expert (for example, the doctor), and may transmit the received confirmation information to the ultrasound apparatus 3000.

According to the present example, when receiving the confirmation message, the ultrasound apparatus 3000 may represent that the ultrasound image displayed on the screen is confirmed by the medical expert (for example, the doctor).

According to examples of the present disclosure, an order of performing operations S1010 to S1060 may be changed, and some of the operations may be omitted.

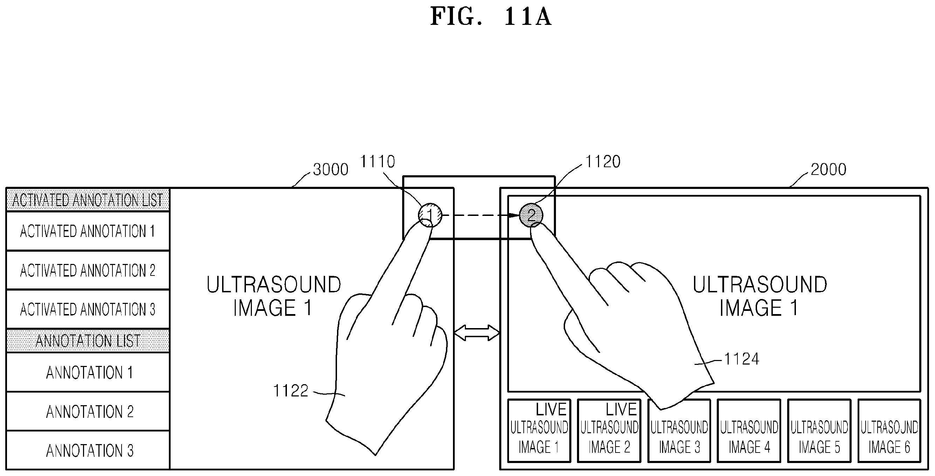

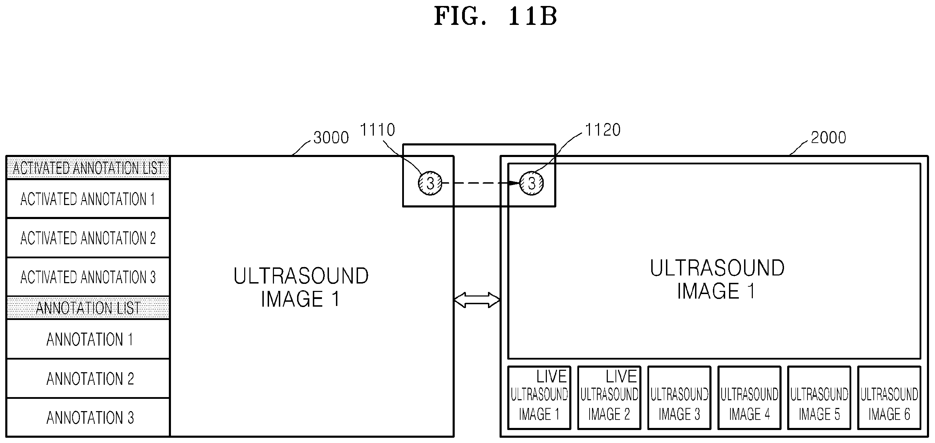

Hereinafter, a method of confirming the second ultrasound information will be described in more detail with reference to FIG. 11 .

FIG. 11 is a diagram showing a GUI for confirming the ultrasound image according to the example of the present disclosure. It is assumed that the second device 2000 is the medical expert device.

As shown in FIG. 11 , the ultrasound apparatus 3000 and the medical expert device (second device) 2000 may respectively include confirmation buttons 1110 and 1120 for confirming the ultrasound image. The confirmation buttons 1110 and 1120 of the present example may be a kind of GUI.

Also, the confirmation button 1110 and 1120 of the present example may display the confirmation status by using a predetermined color, a predetermined number, and a predetermined pattern. For example, if the sonographer may request confirmation of an 'ultrasound image 1', the confirmation button 1110 displayed on the ultrasound apparatus 3000 may be a 'red ① button'. In addition, if the sonographer has requested confirmation of the 'ultrasound image 1' and the medical expert (for example, the doctor) has to confirm the 'ultrasound image 1', the confirmation button 1120 displayed on the medical expert device 2000 may be a 'blue ② button'. Also, when the medical expert (for example, the doctor) selects the blue confirmation button 1120 to confirm the 'ultrasound image 1', the confirmation button 1110 displayed on the ultrasound apparatus 3000 and the confirmation button 1120 displayed on the medical expert device 2000 may be changed to 'green ③ buttons'.

According to another example of the present disclosure, the sonographer may additionally confirm the 'ultrasound image 1' after the medical expert (for example, the doctor) may confirm the 'ultrasound image 1' first. Here, when confirmations of both the medical expert (for example, the doctor) and the sonographer in regard to the 'ultrasound image 1' are completed, the confirmation button 1110 displayed on the ultrasound apparatus 3000 and the confirmation button 1120 displayed on the medical expert device 2000 may be changed to the 'green ③ buttons'.

According to the present example, since the ultrasound image used to diagnose the disease is confirmed only when both the sonographer and the medical expert (for example, the doctor) confirm the ultrasound image, confirmation of a wrong ultrasound image due to a mistake of the sonographer or the medical expert (for example, the doctor) may be prevented.

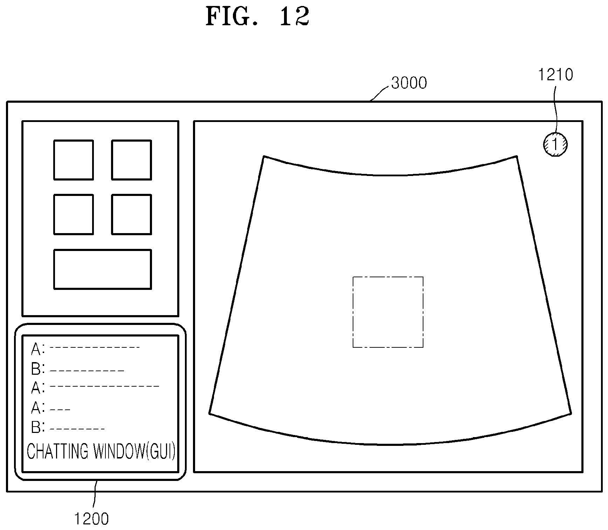

FIG. 12 is a diagram showing a screen on which a chatting window is provided by the ultrasound apparatus 3000, according to an example of the present disclosure.

As shown in FIG. 12 , the ultrasound apparatus 3000 may provide a chatting window 120 through which communication with the medical expert having the second device 2000 may be performed. The ultrasound apparatus 3000 of the present example may receive a user input about the ultrasound image (for example, a question of the sonographer) via the chatting window 1200. In addition, the ultrasound apparatus 3000 may transmit the user input (for example, the question of the sonographer) to the second device 2000, and may receive a response message with respect to the user input from the second device 2000.

For example, the ultrasound apparatus 3000 may receive inquiry information about a status of the examinee (for example, arrhythmia, dyspnoea, and posture of the examinee) during the examination through the chatting window 1200 from the sonographer, and may transmit the inquiry information about the status of the examinee to the second device 2000. In addition, the ultrasound apparatus 3000 receives an order of the medical expert about an action that has to be performed from the second device 2000 in consideration of the status of the examinee, and may display the action ordered by the medical expert (for example, the doctor) on the chatting window 1200.

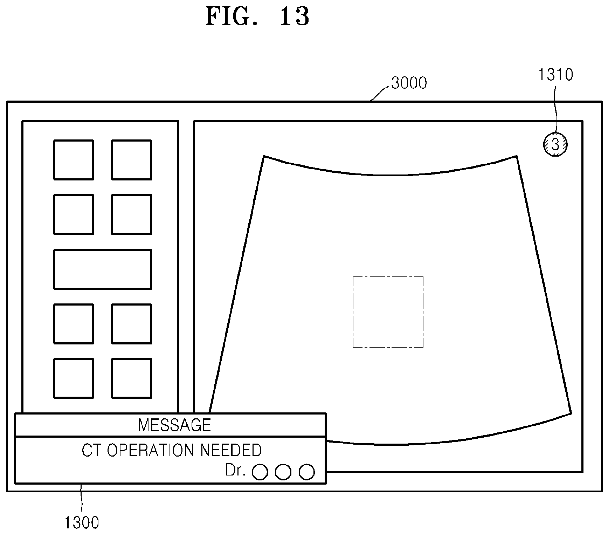

FIG. 13 is a diagram showing a screen displaying a message in the ultrasound apparatus 3000, according to an example of the present disclosure.

The ultrasound apparatus 3000 of the present example may receive a message about the ultrasound image from the second device 2000. In addition, the ultrasound apparatus 3000 may display the received image on the screen.

Here, as shown in FIG. 6 , the ultrasound apparatus 3000 may display the message (for example, a message 'CT is necessary') transmitted from the second device 2000 as a pop-up window 1300.

According to the present example, the ultrasound apparatus 3000 may receive a voice message, in addition to a text message. In this case, the ultrasound apparatus 3000 may output the voice message through a speaker.

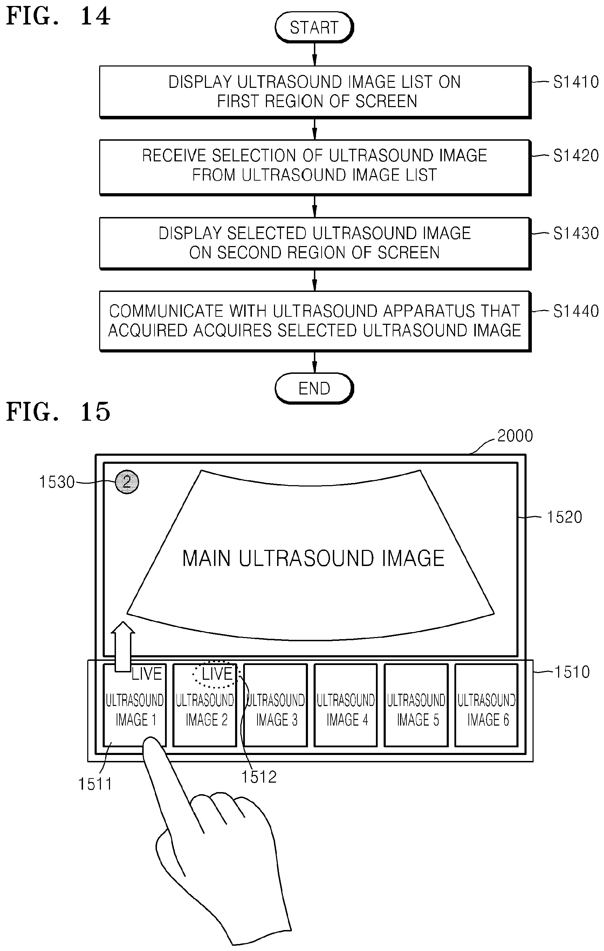

FIG. 14 is a flowchart illustrating a communication method of the medical expert device 2000, according to an example of the present disclosure.

In operation S1410, the medical expert device 2000 may display an ultrasound image list on a first region of the screen. For example, the medical expert device 2000 may display an ultrasound image list including at least one ultrasound image acquired by at least one ultrasound apparatus on the first region of the screen. The ultrasound image list according to the present example may include thumbnail images of the ultrasound images.

In addition, the ultrasound image list of the present example may include real-time ultrasound images transmitted from at least one ultrasound apparatus, and ultrasound images that are stored in advance. The real-time ultrasound image may denote an ultrasound image acquired by the ultrasound apparatus 3000 within a predetermined time period prior to the current time or the time point when the ultrasound image is received by the medical expert device 2000. For example, the real-time ultrasound image may be an image acquired by the ultrasound apparatus 3000 within 10 minutes from the time point when the ultrasound image is received by the medical expert device 2000. That is, when the predetermined time is 10 minutes, the ultrasound image acquired 3 minutes earlier is a real-time image, whereas the ultrasound image acquired 15 minutes earlier may not be a real-time image.

The ultrasound image stored in advance may be an image acquired by the ultrasound apparatus 3000 prior to the predetermined time period and stored. According to the present example, the ultrasound image may be stored in the ultrasound apparatus 3000, the server 4000, and the medical expert device 2000. Therefore, the medical expert device 2000 may read the ultrasound images stored in advance from a memory of the ultrasound apparatus 3000, the server 4000, or the medical expert device 2000.

According to the present example, the medical expert device 2000 may indicate an identification mark on the real-time ultrasound image in order to distinguish the real-time ultrasound image and the ultrasound image stored in advance from each other. For example, the medical expert device 2000 may add an indicator such as 'LIVE' or an exclamation mark (!) to the real-time ultrasound image in the ultrasound image list, make a boundary of the real-time ultrasound image bold, or display the boundary of the real-time ultrasound image in a predetermined color. Also, the medical expert device 2000 may display the real-time ultrasound image to be larger than the ultrasound image stored in advance or may locate the real-time ultrasound image at a center portion in the ultrasound image list.

In operation S1430, the medical expert device 2000 may receive a selection of an ultrasound image from the ultrasound image list. For example, when the medical expert device 2000 includes a touch screen, the medical expert device 2000 senses a medical expert gesture such as a tap, a double-tap, a flicking, or a dragging operation of the ultrasound image included in the ultrasound image list to receive a selection of the medical expert on one ultrasound image from the ultrasound image list.

Otherwise, if the screen on which the ultrasound image list is displayed is not a touch screen, the medical expert (for example, the doctor) may select one ultrasound image from the ultrasound image list by using a mouse or a trackball, or by a voice input.

In operation S1440, the medical expert device 2000 may display the selected ultrasound image on a second region of the screen. Here, the medical expert device 2000 of the present example may display the selected ultrasound image on the second region in a predetermined size. That is, the medical expert device 2000 may display the ultrasound image to be greater than the thumbnail image included in the ultrasound image list on the second region.

In addition, the medical expert device 2000 according to the present example may further display at least one of the analysis information of the sonographer about the ultrasound image displayed on the second region, measurement information of the ultrasound image displayed on the second region, and the examinee information according to the sharing level thereof.

In operation S1440, the medical expert device 2000 may communicate with the ultrasound apparatus 3000 that acquires the selected ultrasound image.

For example, the medical expert device 2000 according to the present example may provide a chatting window for communicating with the ultrasound apparatus 3000 on a third region of the screen. In addition, the medical expert device 2000 may receive an input of the medical expert (for example, the doctor) about the ultrasound image displayed on the second region, through the chatting window. Here, the medical expert device 2000 may transmit the input of the medical expert received through the chatting window to the ultrasound apparatus 3000 in a chatting session.

Also, the medical expert device 2000 according to the present example may receive a request for confirming the selected ultrasound image from the ultrasound apparatus 3000. Then, the medical expert device 2000 may transmit the confirmation information to the ultrasound apparatus 3000 when the confirmation information about the selected ultrasound image is input.

In addition, the medical expert device 2000 of the present example may transmit control information for controlling the ultrasound apparatus 3000 to the ultrasound apparatus 3000. The control information of the present example may include at least one of a control command for selecting and displaying another ultrasound image that is different from the ultrasound image displayed on the second region, a control command for expanding or reducing the ultrasound image displayed on the second region, a control command for storing the ultrasound image displayed on the second region, a control command for 3D rendering the ultrasound image displayed on the second region, a control command for adding an annotation or a body marker to the ultrasound image displayed on the second region, a control command for measuring a region of interest, and a control command for correcting analysis information of the ultrasound image displayed on the second region.

Hereinafter, a screen for displaying a plurality of ultrasound images acquired by a plurality of ultrasound apparatuses in the medical expert device 2000 will be described with reference to FIGS. 15 through 18 .

FIG. 15 is a diagram showing a screen providing an ultrasound image list by the medical expert device 2000, according to an example of the present disclosure.

As shown in FIG. 15 , the screen of the medical expert device 2000 according to the present example may be classified as a first region 1510 for displaying an ultrasound image list and a second region 1520 displaying a main ultrasound image.

The ultrasound image list may include the real-time ultrasound images acquired by the ultrasound apparatus 3000 and the ultrasound images stored in advance. Here, the medical expert device 2000 of the present example may distinguish the ultrasound images stored in advance and the real-time ultrasound images from each other by adding a mark 'LIVE' 1512 to the real-time ultrasound images. For example, an 'ultrasound image 1' 1511 and an ultrasound image 2 marked with 'LIVE' 1512 are the real-time ultrasound images, and an ultrasound image 3, an ultrasound image 4, an ultrasound image 5, and an ultrasound image 6 may be the ultrasound images that are stored in advance.

According to the present example, when a touch-and-drag input, in which the medical expert drags the ultrasound image 1 1511 to the second region 1520 while touching the ultrasound image 1 1511, is sensed, the medical expert device 2000 may display the ultrasound image 1 1511 on the second region 1520 as a main ultrasound image. FIG. 15 shows the touch-and-drag gesture as an example of selecting the ultrasound image; however, the examples of the present disclosure are not limited thereto. For example, the doctor may select the ultrasound image by using a tap gesture, a double tap gesture, a voice input, or a physical selection button.

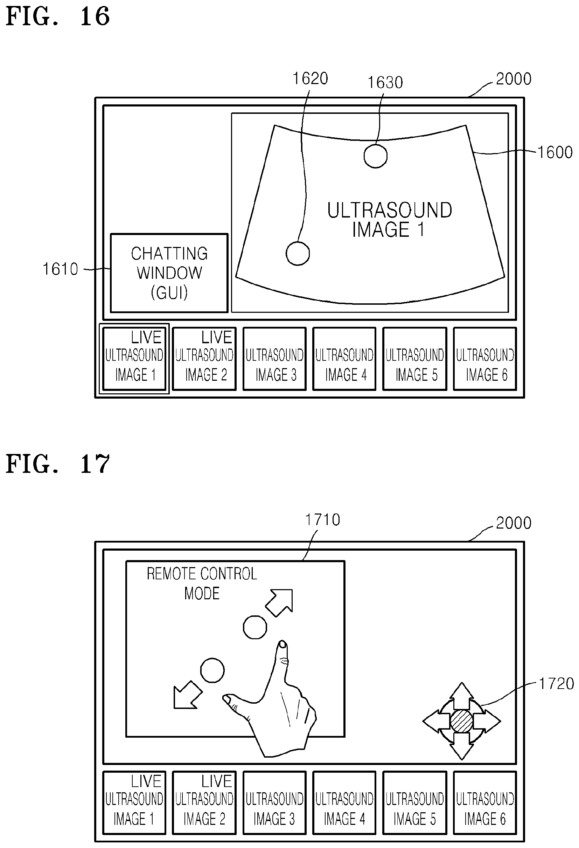

The medical expert device 2000 of the present example communicates with the ultrasound apparatus 3000 that acquires the ultrasound image 1 1511. This will be described later with reference to FIG. 16 .

In addition, the medical expert device 2000 may display a GUI for receiving a confirmation input of the ultrasound image 1 1511, that is, a confirmation button 1530, on the screen. The medical expert device 2000 may receive a confirmation input of the medical expert about the ultrasound image 1 1511 via the confirmation button 1530. When the medical expert selects the confirmation button 1530 to confirm the ultrasound image 1 1511, a color or a pattern of the confirmation button 1530 may be changed. In addition, the medical expert device 2000 may transmit a confirmation message of the ultrasound image 1 1511 to the ultrasound apparatus 3000 that acquires the ultrasound image 1 1511.

FIG. 16 is a diagram showing a screen for displaying a chatting window and a pointer in the medical expert device 2000, according to an example of the present disclosure. In FIG. 16 , it is assumed that the medical expert is a doctor.

As shown in FIG. 16 , the medical expert device 2000 of the present example may provide a chatting window 1610 for communicating with the ultrasound apparatus 3000 on a third region of the screen. In addition, the medical expert device 2000 may receive an input of the doctor about an ultrasound image 1 1600 via the chatting window 1610, and may transmit the received input to the ultrasound apparatus 3000.

That is, the medical expert device 2000 of the present example may receive an image message, a voice message, and a text message of the sonographer from the ultrasound apparatus 3000, and may transmit an image message, a voice message, and a text message of the medical expert to the ultrasound apparatus 3000 via the chatting window 1610.

For example, the doctor may identify a result report generated by the sonographer after performing the ultrasound examination and may confirm that the report may be finished in the current status of the examined status, or may input a message requesting correction of a result value of the report in the chatting window 1610.

Also, if the ultrasound image is not exact and measurement information with respect to the ultrasound image is small, a message requesting a re-examination may be input to the chatting window 1610. The doctor may request the sonographer to perform an examination such as CT or MRI.

In addition, since the measured values may vary depending on the status of the examinee, the doctor may inquire about the sonographer's opinion regarding the measurement values or may ask the sonographer about an operation status of the examinee via the chatting window 1610.

According to the present example, the medical expert device 2000 may display a pointer 1630 of the ultrasound apparatus 3000 and a pointer 1620 of the medical expert device 2000 on the ultrasound image 1 1600. Here, the medical expert device 2000 may receive information about a location of the pointer 1620 of the ultrasound apparatus 3000 from the ultrasound apparatus 3000.

Also, the ultrasound apparatus 3000 may display the pointer 1630 of the ultrasound apparatus 3000 and the pointer 1620 of the medical expert device 2000 on the ultrasound image 1 1600. Therefore, the sonographer and the doctor may identify areas of interest of each other by locating the pointer on a point of interest during the chatting.

FIG. 17 is a diagram showing an example of a GUI for remotely controlling the ultrasound apparatus 3000 by the medical expert device 2000, according to an example of the present disclosure.

The medical expert device 2000 of the present example may sense a pinch input on a main ultrasound image 1710. In addition, the medical expert device 2000 may transmit a control command for expanding or reducing the main ultrasound image 1710 to the ultrasound apparatus 3000 based on the pinch input.

For example, when the user touches the screen with two fingers and increases a distance between the two fingers, a control command for expanding the main ultrasound image 1710 according to the distance between the fingers is transmitted to the ultrasound apparatus 3000. Otherwise, when the user touches the screen with two fingers and reduces the distance between the two fingers, a control command for reducing the main ultrasound image 1710 may be transmitted to the ultrasound apparatus 3000.

When the main ultrasound image 1710 displayed on the medical expert device 2000 is expanded or reduced in the medical expert device 2000, a resolution of the ultrasound image may be lowered. Thus, the medical expert device 2000 remotely controls the ultrasound apparatus 3000 to expand or reduce the main ultrasound image 1710 and to transmit the expanded or reduced ultrasound image to the medical expert device 2000 in real-time.

In addition, according to the present example, the medical expert device 2000 may transmit a control command for adjusting a direction of a probe to the ultrasound apparatus 3000 based on the medical expert's input. For example, the medical expert device 2000 may remotely control the probe direction of the ultrasound apparatus 3000 according to the medical expert's input for moving a remote control button 1720 displayed on the screen to an upper/lower/left/right position, or another position therebetween.

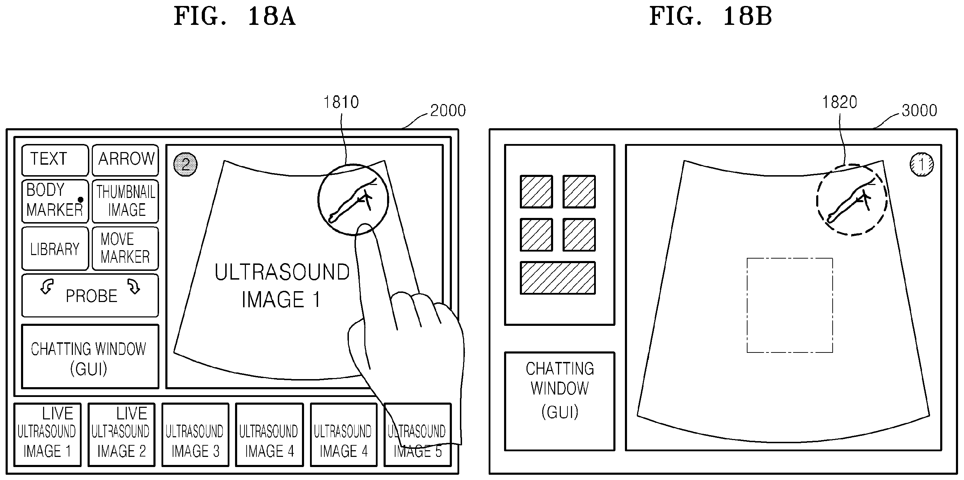

FIGS. 18A and 18B are diagrams showing an example of a GUI for adding a body marker to the ultrasound image remotely by the medical expert device 2000, according to an example of the present disclosure.

The body marker according to the present example may be a figure for identifying a location to which an ultrasound wave is scanned or an object. The body marker of the present example may include a figure representing an object to which the ultrasound wave is scanned and a figure representing a location of the probe contacting the object. An example of the body marker may be an arm shape, a liver shape, or a uterus shape.

As shown in FIG. 18A , the medical expert device 2000 may receive an input of the medical expert for adding a body marker 1810 to the ultrasound image. In this case, the medical expert device 2000 may transmit to the ultrasound apparatus 3000 a control command for adding the body marker 1810 selected by the medical expert to the ultrasound image displayed on the ultrasound apparatus 3000.

As shown in FIG. 18B , the ultrasound apparatus 3000 receives the control command for adding the body marker from the medical expert device 2000, and may add a body marker 1820 to the ultrasound image displayed on the screen thereof.

According to the present example, the medical expert (for example, a doctor) may control the ultrasound apparatus 3000 via the medical expert device 2000 from a remote distance.

FIGS. 18A and 18B show an example of a GUI for adding the body marker; however, the examples of the present disclosure are not limited thereto. For example, the medical expert device 2000 may remotely control the ultrasound apparatus 3000 to add an annotation, to re-measure the region of interest, or to perform 3D image rendering.

Also, the medical expert device 2000 may control the ultrasound apparatus 3000 to display a certain ultrasound image, and thus, the medical expert (for example, the doctor) may select and display a desired ultrasound image from the thumbnail images or review window displayed on the ultrasound apparatus 3000, and then, check the selected ultrasound image. Also, the medical expert (for example, the doctor) may expand another ultrasound image that seems to be necessary while reviewing the selected ultrasound image.

In addition, the medical expert (for example, the doctor) may control the ultrasound apparatus 3000 remotely to store the necessary ultrasound image again, or may directly correct the report generated by the sonographer.

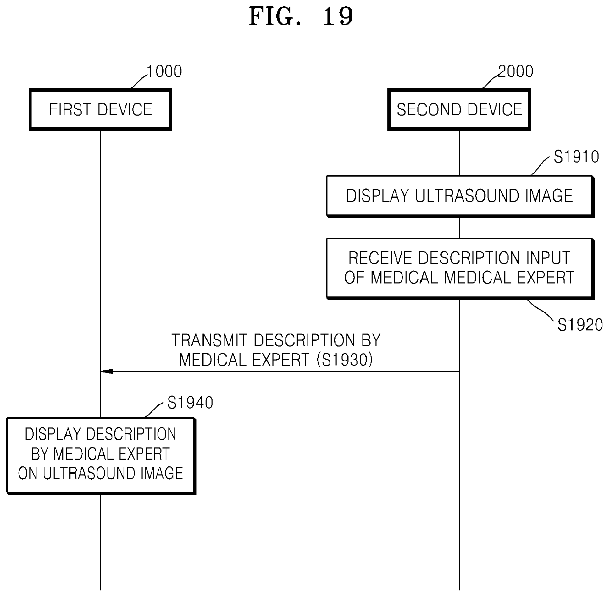

FIG. 19 is a flowchart illustrating a communication method between the medical expert device 2000 and the examinee device 1000, according to an example of the present disclosure. In FIG. 19 , it is assumed that the second device 2000 is the medical expert device, and the second device 1000 is the examinee device.

In operation S1910, the second device 2000 may display an ultrasound image on the screen thereof. Here, according to the present example, the ultrasound image displayed on the second device 2000 may be an ultrasound image acquired from the ultrasound apparatus 3000 in real-time, or an ultrasound image stored in a memory in advance.

In operation S1920, the second device 2000 may receive a description by the medical expert about the ultrasound image displayed on the screen. For example, the description by the medical expert may include representing a region of interest (for example, a tumor, an embryo, a head of the embryo, a hand of the embryo, etc.), a description about the region of interest, an analysis result of the measurement value (for example, head girth, the number of fingers of an embryo, etc.), or existence of lesions; however, the examples of the present disclosure are not limited thereto.

In operation S1930, the second device 2000 may transmit the description of the medical expert to the first device 1000. Here, the first device 1000 may be an examinee device displaying the ultrasound image that is the same as that displayed on the second device 2000.

According to the present example, the second device 2000 may transmit the description by the medical expert to the first device 1000 through wired or wireless communication. Otherwise, the second device 2000 may transmit the description by the medical expert to the first device 1000 via the server 4000.

According to the present example, the second device 2000 may transmit the description by the medical expert about the ultrasound image input from the medical expert to the ultrasound apparatus 3000. Here, the second device 2000 may transmit the description by the medical expert simultaneously to both the first device 1000 and the ultrasound apparatus 3000, or may transmit them sequentially.

In operation S1940, the first device 1000 may display the description by the medical expert on the ultrasound image. That is, the first device 1000 may receive the description by the medical expert about the ultrasound image displayed on the screen, and then, may display the description by the medical expert on the ultrasound image.

According to the present example, the description by the medical expert may be displayed on the first device 1000 in real-time or may be stored in the first device 1000. Here, displaying of the description by the medical expert on the first device 1000 in real-time denotes that the description by the medical expert is displayed on the first device 1000 within a predetermined time period after the input of the description to the second device 2000.

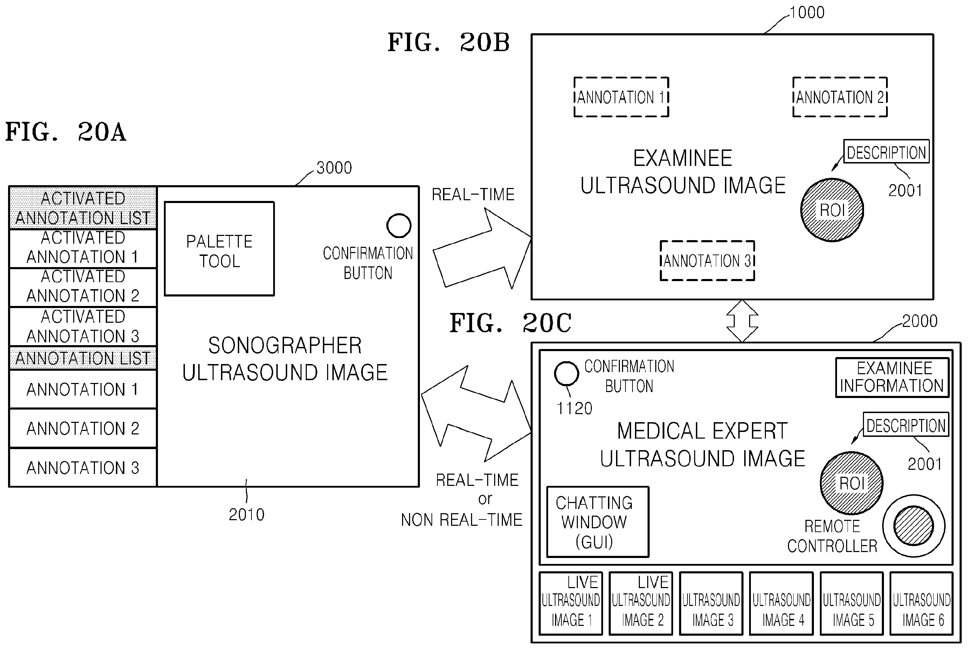

FIGS. 20A through 20C are diagrams showing screens of the ultrasound apparatus 3000, the first device 1000, and the second device 2000, according to an example of the present disclosure.

As shown in FIG. 20A , the ultrasound apparatus 3000 may transmit the ultrasound image displayed on the screen thereof to the first device 1000 and the second device 2000 in real-time. In addition, the ultrasound apparatus 3000 may display a GUI for adding an annotation or color to the ultrasound image for the user of the first device 1000. The ultrasound apparatus 3000 may display a confirmation button to request the confirmation of the ultrasound image to the second device 2000.

In addition, the ultrasound image displayed on the screen that the user (for example, the sonographer) of the ultrasound apparatus 3000 watches may be displayed on the first device 1000 and the second device 2000 in real-time. Here, the information about the ultrasound image displayed on the first device 1000 and the second device 2000 may vary depending on the sharing levels of the first device 1000 and the second device 2000.

For example, as shown in FIG. 20B , the ultrasound image, the annotation for describing the ultrasound image, and the identification mark of the region of interest included in the ultrasound image may be displayed on the screen of the first device 1000 according to the sharing level of the first device 1000.

On the other hand, as shown in FIG. 20C , the ultrasound image, the information about the examinee, the measurement information with respect to the ultrasound image, the chatting window for communicating with the user of the ultrasound apparatus 3000, the confirmation button for confirming the ultrasound image, and the GUI for remotely controlling the ultrasound apparatus 3000 may be displayed on the screen of the second device 2000 according to the sharing level of the second device 2000.

In addition, the second device 2000 may display information about a non-real time ultrasound image, as well as the real-time ultrasound image, on the screen thereof. For example, the second device 2000 displays the ultrasound image list including the real-time ultrasound image and the ultrasound image that is stored in advance on the first region of the screen, and may display one ultrasound image selected from the ultrasound image list on the second region of the screen.

According to the present example, as shown in FIG. 20C , when the user (for example, the medical expert) of the second device 2000 inputs a description 2001 about the ultrasound image, the second device 2000 may transmit the input description 2001 to the first device 1000. Here, as shown in FIG. 20B , the first device 1000 may display the description 2001 transmitted from the second device 2000 on the ultrasound image.

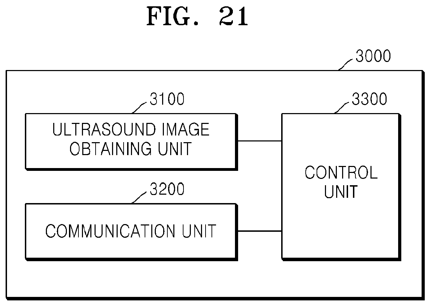

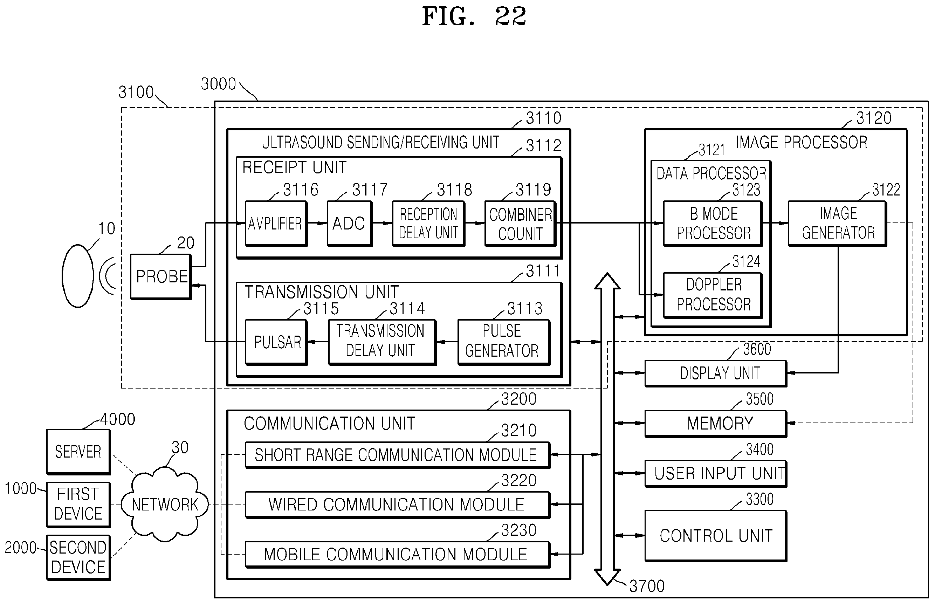

FIGS. 21 and 22 are block diagrams showing an ultrasound apparatus 3000, according to an example of the present disclosure.

As shown in FIG. 21 , the ultrasound apparatus 3000 of the present example may include an ultrasound image obtaining unit 3100, a communication unit 3200, and a control unit 3300. However, not all the components shown in FIG. 21 may be essential elements. The ultrasound apparatus 3000 may include the illustrated elements and other elements, or may include only some of the illustrated elements. For example, as shown in FIG. 22 , the ultrasound apparatus 3000 according to another example of the present disclosure may further include a user input unit 3400, a memory 3500, and a display unit 3600, in addition to the ultrasound image obtaining unit 3100, the communication unit 3200, and the control unit 3300. The above components may be connected to each other via a bus 3700.

Hereinafter, the above components will be described.

The ultrasound image obtaining unit 3100 of the present disclosure may acquire ultrasound image data about an object. The ultrasound image data may be 2D ultrasound image data or 3D ultrasound image data about the object.

According to the present example, the ultrasound image obtaining unit 3100 may include a probe 20, an ultrasound sending/receiving unit 3110, and an image processor 3120.

The probe 20 sends an ultrasound signal to an object 10 according to a driving signal applied from the ultrasound sending/receiving unit 3110, and receives an echo signal reflected by the object 10. The probe 20 may include a plurality of transducers that vibrate according to an electric signal to generate the ultrasound that is acoustic energy. Also, the probe 20 may be connected to a main body of the ultrasound apparatus 3000 via a wire or wirelessly, and the ultrasound apparatus 3000 may include a plurality of probes 20 according to the present example. The probe 20 may include at least one of a 1-dimensional (1D) probe, a 1.5-dimensional (1.5D) probe, a 2D (matrix) probe, and a 3D probe.

A transmission unit 3111 applies a driving signal to the probe 20, and may include a pulse generator 3113, a transmission delay unit 3114, and a pulsar 3115. The pulse generator 3113 generates pulses for forming a transmission ultrasound according to a predetermined pulse repetition frequency (PRF), and the transmission delay unit 3114 applies a delay time for determining transmission directionality to the pulses. Each of the pulses to which the delay time is applied corresponds to each of a plurality of piezoelectric vibrators included in the probe 20. The pulsar 3115 applies the driving signal (or the driving pulse) to the probe 20 at timings corresponding to the pulses to which the delay time is applied.

A receipt unit 3112 processes an echo signal transmitted from the probe 20 to generate ultrasound data, and may include an amplifier 3116, an analog-to-digital converter (ADC) 3117, a receipt delay unit 3118, and a combiner 3119. The amplifier 3116 amplifies the echo signal for each of channels, and the ADC 3117 converts the amplified echo signal. The receipt delay unit 3118 applies a delay time for determining a reception directionality to the echo signal that is converted to a digital signal, and the combiner 3119 combines echo signals processed by the receipt delay unit 3118 to generate the ultrasound data.

The image processor 3120 generates ultrasound images by performing a scan conversion of the ultrasound data generated by the ultrasound sending/receiving unit 3110. The ultrasound image may include a gray scale image obtained by scanning the object in an amplitude (A) mode, a brightness (B) mode, and a motion (M) mode, and a Doppler image representing a moving object by using the Doppler effect. The Doppler image may include a bloodstream Doppler image (or color Doppler image) representing flow of blood, a tissue Doppler image representing movement of tissues, and a spectral Doppler image representing velocity of the object as a waveform.

A B mode processor 3123 extracts B mode components from the ultrasound data, and processes the B mode components. An image generator 3122 may generate an ultrasound image in which an intensity of a signal is represented by brightness based on the B mode components extracted by the B mode processor 3123.

Likewise, a Doppler processor 3124 extracts Doppler components from the ultrasound data, and the image generator 3122 may generate a Doppler image in which movement of the object is represented by a color or a waveform based on the extracted Doppler components.

The image generator 3122 according to the present example may generate a 3D ultrasound image by performing a volume rendering process of volume data, and may generate an elastic mode image in which a transformation degree of the object 10 according to a pressure is imaged.

Moreover, the image generator 3122 may represent various pieces of additional information as a text or a graphic on the ultrasound image. For example, the image generator 3122 may add at least one annotation relating to all or some of the ultrasound image to the ultrasound image. That is, the image generator 3122 may analyze the ultrasound image, and may recommend at least one annotation relating to all or some of the ultrasound image based on a result of the analysis. Also, the image generator 3122 may add at least one annotation selected by the user to the ultrasound image.

In addition, the image processor 3120 may extract a region of interest from the ultrasound image by using an image processing algorithm. Here, the image processor 3120 may add a color, a pattern, or a boundary to the region of interest.

The communication unit 3200 may include one or more components enabling communications between the ultrasound apparatus 3000 and the first device 1000, the ultrasound apparatus 3000 and the second device 2000, and the ultrasound apparatus 3000 and the server 4000. For example, the communication unit 3200 may include a short distance communication module 3210, a wired communication module 3220, a mobile communication module, etc.

The short distance communication module 3210 refers to a module for short distance communication. As a short distance communication technology, WLAN (e.g., Wi-Fi), Bluetooth, BLE, UWB, ZigBee, NFC, WFD, Infrared Data Association (IrDA), etc. may be used.

The wired communication module 3220 is a communication module using an electric signal or an optical signal. As a wired communication technology of the present example, a pair cable, a coaxial cable, an optical fiber cable, an Ethernet cable, etc. may be used.

The mobile communication module transmits/receives wireless signals to/from at least one of a base station, an external device, and a server on a mobile communication network. Here, the wireless signal may include various types of data according to a voice call signal, a video call signal, or text/multimedia message transmission.

The communication unit 3200 is connected to a network 30 by using a wire or wirelessly to communicate with an external device (for example, the first device 1000 or the second device 2000) or the server 4000. The communication unit 3200 may transmit/receive data to/from a hospital server or other medical equipment in the hospital, which are connected to the communication unit 3200 via a picture archiving and communication system (PACS). Also, the communication unit 3200 may perform data communication according to digital imaging and communications in medicine (DICOM).

The communication unit 3200 may transmit data relating to diagnosis of the object, for example, ultrasound images, ultrasound data, Doppler data, etc. of the object 10, via the network 30, and may receive medical images captured by other medical apparatuses such as a CT, an MRI, an X-ray, etc. In addition, the communication unit 3200 may receive information about a medical history or treatment schedule of an examinee from the server 4000, to be used in the diagnosis of the object 10.

The communication unit 3200 may transmit first ultrasound information about the ultrasound image to the first device 1000 and second ultrasound information about the ultrasound image to the second device 2000 according to the sharing levels that are set in advance.

The communication unit 3200 may receive a message about the ultrasound image from the second device 2000. Here, the message may include a voice message, a text message, or a video message. The communication unit 3200 may transmit a user input to the second device 2000, and may receive a response message from the second device 2000.

According to the present example, the communication unit 3200 may transmit a request for confirmation of the second ultrasound information to the second device 2000, and may receive a confirmation message of the second ultrasound information from the second device 2000.

In addition, the communication unit 3200 may receive control information from the second device 2000. Here, the control information may include at least one of a control command for selecting and displaying a different ultrasound image from a currently displayed image, a control command for expanding or reducing the ultrasound image, a control command for storing the ultrasound image, a control command for 3D rendering the ultrasound image, a control command for adding an annotation or a body marker, a control command for measuring a region of interest, and a control command for correcting analysis information of the ultrasound image; however, the examples of the present disclosure are not limited thereto.

The communication unit 3200 may request the server 4000 for authentication of the first device 1000 and the second device 2000, and if the authentication is made, the communication unit 3200 may receive sharing level information of the first device 1000 and the second device 2000 from the server 4000.

The control unit 3300 controls overall operations of the ultrasound apparatus 3000. That is, the control unit 3300 may control the ultrasound image obtaining unit 3100, the communication unit 3200, the user input unit 3400, the memory 3500, and the display unit 3600.

For example, the control unit 3300 may identify the sharing level of each of the first and second devices 1000 and 2000 for sharing the ultrasound image. In addition, the control unit 3300 may generate first ultrasound information corresponding to a first sharing level of the first device 1000, and second ultrasound information corresponding to a second sharing level of the second device 2000.

When a touch input that touches the annotation displayed on the ultrasound image for a predetermined time period or longer is sensed, the control unit 3300 may enable the touched annotation to move. The control unit 3300 may select a region of interest from the ultrasound image, and may indicate an identification mark on the region of interest.

The control unit 3300 may execute a control command corresponding to the control information transmitted from the second device 2000.

The user input unit 3400 is a unit used by the user (for example, the sonographer) to input data for controlling the ultrasound apparatus 300. For example, the user input unit 3400 may be a keypad, a dome switch, a touch pad (a contact capacitance type, a pressure resistive type, an infrared sensing type, a surface ultrasound transfer type, an integral tension measurement type, a piezo effect type, etc.), a trackball, or a jog switch; however, the examples of the present disclosure are not limited thereto. That is, the user input unit 3400 may further include an electrocardiogram (ECG) measuring module, a respiratory sensor module, a voice recognition sensor, a gesture recognition sensor, a fingerprint recognition sensor, an iris recognition sensor, a depth sensor, a distance sensor, etc.

The user input unit 3400 of the present example may detect a proximity touch, as well as a real-touch. The user input unit 3400 may sense a touch input on the ultrasound image (for example, a touch-and-hold, a tap, a double tap, or a flicking operation). Also, the user input unit 3400 may sense a drag input from a point where a touch input is sensed. In addition, the user input unit 3400 may sense a multiple touch input (for example, a pinch operation) on at least two or more points included in the ultrasound image.

For example, the user input unit 3400 may receive a selection of at least one annotation included in the annotation list displayed on the screen. Also, the user input unit 3400 may receive a drag & drop input that involves dragging the selected annotation to the region where the ultrasound image is displayed and dropping the annotation, and may sense a drag input of the user onto the annotation displayed on the ultrasound image.

In addition, the user input unit 3400 may receive a user input about the ultrasound image through the chatting window.

The memory 3500 may store programs for processing and controlling the control unit 3300, and may store input/output data (for example, the annotation list set in advance, the ultrasound images, the examinee information, probe information, body markers, etc.).

The memory 3500 may include at least one storage medium from among a flash memory, a hard disk, a micro multimedia card, a card-type memory (e.g., a security digital (SD) or XD memory), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. Also, the ultrasound apparatus 3000 may operate web storage or a cloud server for performing the storing function of the memory 3500 on the Internet.

The display unit 3600 displays and outputs the information processed by the ultrasound apparatus 3000. For example, the display unit 3600 displays the ultrasound images, or may display a user interface (UI) or GUI related to a control panel. In addition, the display unit 3600 may display a list of devices that may communicate with the ultrasound apparatus 3000, an annotation list about the plurality of annotations that are set in advance, and an activated annotation list about the annotations displayed on the screen.

According to the present example, the display unit 3600 may display an annotation selected from the annotation list on the ultrasound image. For example, the display unit 3600 may display the selected annotation based on the drag & drop input about the annotation included in the annotation list.

The display unit 3600 may move the displayed location of the annotation according to the drag input of the user. Also, the display unit 3600 may display an activated annotation list about at least one annotation added to the ultrasound image on a predetermined region of the screen.

The display unit 3600 of the present example may display a message received from the second device 2000 and may provide a chatting window for communicating with the second device 2000 on the screen thereof.

If a display panel and a touchpad are layered to form a touchscreen, the display unit 3600 may be used as an input device as well as an output device. The display unit 3600 may include at least one of a liquid crystal display (LCD), a thin film transistor-liquid crystal display (TFT-LCD), an organic light-emitting diode (OLED), a flexible display, a 3D display, and an electrophoretic display. According to the form of the ultrasound apparatus 3000, the ultrasound apparatus 3000 may include two or more display units 3600.

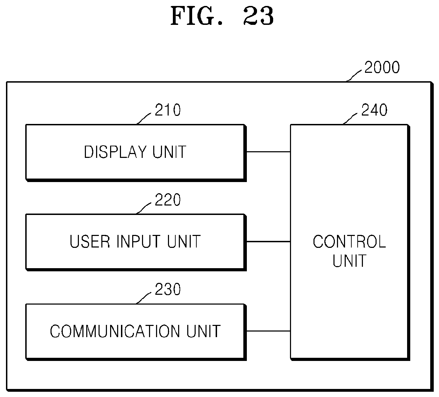

FIG. 23 is a block diagram showing a medical expert device 2000 according to an example of the present disclosure.

As shown in FIG. 23 , the medical expert device 2000 of the present example may include a display unit 210, a user input unit 220, a communication unit 230, and a control unit 240. However, not all the components shown in FIG. 23 are essential elements. That is, the medical expert device 2000 may include more or less components than those of FIG. 23 .

Also, the medical expert device 2000 may be realized as various types. For example, the medical expert device 2000 may be realized as a mobile terminal, as well as a fixed terminal. Examples of the mobile terminal may include a laptop computer, a personal digital assistant (PDA), a tablet PC, etc.

Hereinafter, the above components will be described.

The display unit 210 displays and outputs information processed by the medical expert device 2000. For example, the display unit 210 may display an ultrasound image list including at least one ultrasound image acquired by at least one ultrasound apparatus 3000 on a first region of the screen, and may display an ultrasound image selected from the ultrasound image list on a second region of the screen. Here, the ultrasound image list of the present example may include real-time ultrasound images transmitted from at least one ultrasound apparatus and ultrasound images stored in advance. According to another example, the ultrasound image list may include real-time ultrasound images transmitted from the ultrasound apparatus 3000 only, or ultrasound images stored in advance.

The display unit 210 may display at least one of analysis information of the sonographer about the ultrasound image, measurement information with respect to the ultrasound image, and examinee information, in addition to the ultrasound image, according to a sharing level of the medical expert device 2000.

Also, the display unit 210 may indicate a pointer of the ultrasound apparatus 3000 and a pointer of the medical expert device 2000, and may provide a chatting window for communicating with the ultrasound apparatus 3000 on a third region of the screen. In addition, the display unit 210 may display a GUI for receiving a confirmation input about the ultrasound image from the medical expert (for example, a doctor).

The user input unit 220 is a unit for inputting data for controlling the medical expert device 2000 from the medical expert (for example, the doctor). For example, the user input unit 220 may be a keypad, a dome switch, a touch pad (a contact capacitance type, a pressure resistive type, an infrared sensing type, a surface ultrasound transfer type, an integral tension measurement type, a piezo effect type, etc.), a trackball, or a jog switch; however, the examples of the present disclosure are not limited thereto. In particular, if a touch pad is layered with a display panel, this is referred to as a touch screen.

The user input unit 220 of the present example may detect a proximity touch, as well as a real-touch. The user input unit 220 may sense a touch input on the ultrasound image (for example, a touch-and-hold, a tap, a double tap, or a flicking operation). Also, the user input unit 220 may sense a drag input from a point where a touch input is sensed. In addition, the user input unit 220 may sense a multiple touch input (for example, a pinch operation) on at least two or more points included in the ultrasound image.

According to the present example, the user input unit 220 may receive a selection of an ultrasound image from the ultrasound image list. Also, the user input unit 220 may receive an input of the user about the ultrasound image through the chatting window, and may receive a confirmation input about the ultrasound image through the GUI for receiving the confirmation input.

The user input unit 220 may receive a description by the medical expert about the ultrasound image. The description by the medical expert may include representing a region of interest (for example, a tumor, an embryo, a head of the embryo, a hand of the embryo, etc.), a description about the region of interest, an analysis result of the measurement value (for example, head girth, the number of fingers of an embryo, etc.), or existence of lesions; however, the examples of the present disclosure are not limited thereto.

The communication unit 230 may include one or more components enabling communications between the ultrasound apparatus 3000 and the first device 1000, the ultrasound apparatus 3000 and the second device 2000, and the ultrasound apparatus 3000 and the server 4000. For example, the communication unit 230 may include a short distance communication module, a mobile communication module, a wired internet module, a wireless internet module, etc.

The short distance communication module refers to a module for short distance communication. As a short distance communication technology, WLAN (e.g., Wi-Fi), Bluetooth, BLE, UWB, ZigBee, NFC, WFD, IrDA, etc. may be used.

The mobile communication module transmits/receives wireless signals to/from at least one of a base station, an external device, and a server on a mobile communication network. The wireless internet module is a module for accessing wireless internet, and may be built into or provided outside the medical expert device 2000. The wired internet module is a module for accessing wired internet.

The communication unit 230 may communicate with the ultrasound apparatus 3000 that acquires the ultrasound image selected by the medical expert (for example, the doctor). For example, the communication unit 230 may transmit an input about the ultrasound image to the ultrasound apparatus 3000. Also, the communication unit 230 may receive a request for confirmation about the selected ultrasound image from the ultrasound apparatus 3000, and may transmit the confirmation information about the selected ultrasound image to the ultrasound apparatus 3000.

In addition, the communication unit 230 may transmit control information for controlling the ultrasound apparatus 3000 to the ultrasound apparatus 3000. For example, the communication unit 230 may transmit to the ultrasound apparatus 3000 at least one of a control command for selecting and displaying another ultrasound image, a control command for expanding or reducing the ultrasound image, a control command for storing the ultrasound image, a control command for 3D rendering the ultrasound image, a control command for adding an annotation or a body marker, a control command for measuring a region of interest, and a control command for correcting analysis information of the ultrasound image. The communication unit 230 may transmit the description by the medical expert to the first device 1000.

The control unit 240 controls overall operations of the medical expert device 2000. That is, the control unit 240 may control the display unit 210, the user input unit 220, and the communication unit 230.

According to the present example, the control unit 240 may indicate an identification mark on the real-time ultrasound image for distinguishing the real-time ultrasound image from the ultrasound images that are stored in advance.

The above-described present disclosure may be implemented with at least one processor and include a computer readable medium including program instructions for executing various operations realized by a computer. The computer readable medium may include program instructions, a data file, and a data structure, separately or cooperatively. The program instructions and the media may be specially designed and constructed for the purposes of the present disclosure, or they may be of a kind well known and available to one of ordinary skill in the art of computer software arts. Examples of the computer readable media include magnetic media (e.g., hard disks, floppy disks, and magnetic tapes), optical media (e.g., CD-ROMs or DVD), magneto-optical media (e.g., floptical disks), and hardware devices (e.g., ROMs, RAMs, or flash memories, etc.) that are specially configured to store and perform program instructions. The media may also be transmission media such as optical or metallic lines, wave guides, etc. specifying the program instructions, data structures, etc. Examples of the program instructions include both machine code, such as that produced by a compiler, and files containing high-level languages codes that may be executed by the computer using an interpreter.

According to the examples of the present disclosure, the ultrasound apparatus may transmit appropriate ultrasound information to a receiving device according to a sharing level of the receiving device (for example, the examinee device and the medical expert device). Therefore, excessive exposure of the ultrasound information may be prevented. Also, the ultrasound apparatus may provide the examinee device with ultrasound information so that the examinee may easily comprehend, and may provide the medical expert (for example, the doctor) with detailed information necessary for the diagnosis.

In addition, since the user of the ultrasound apparatus may communicate with the medical expert about the ultrasound images in real-time, the medical expert may direct appropriate operations while transferring an examinee in emergency to a hospital. Therefore, if there is an ultrasound apparatus, the examinee may diagnose himself/herself.

While the present disclosure has been particularly shown and described with reference to examples thereof, it will be understood by those of ordinary skill in the art that various changes in form and details may be made therein without departing from the scope of the present invention as defined by the following claims.

For instance, the description above has been focussed on ultrasound imaging. However, the disclosure can be extended to other forms of medical imaging such as radiography, Magnetic Resonance Imaging (MRI), nuclear medicine, elastography, tactile imaging, photoacoustic imaging, thermography, tomography, and echocardiography. Also with these techniques, the specialist responsible for acquiring the images differs from the specialist who interprets the images for the purpose of forming a diagnosis. Information can be exchanged in a manner similar to that described above. Based on a sharing level, different types and quantities of additional information can be exchanged. The additional information is in addition to image information that can be used to reconstruct the image in an external image.

As described above, the ultrasound apparatus determines a sharing level for a first and second external device and transmits information to these devices in accordance with the respective sharing level. The sharing level can be obtained during or following an authentication of the external devices. The same steps can be followed for communication between the first and second device. For instance, the second device, such as a medical expert device, may request authentication of the first device, such as an examinee device and/or the ultrasound apparatus. During or following this authentication, the medical expert device may receive information regarding a sharing level of the examinee device and/or the ultrasound apparatus. The type and/or amount of information to be transmitted to the examinee device or the ultrasound apparatus can differ according to the respective sharing levels.

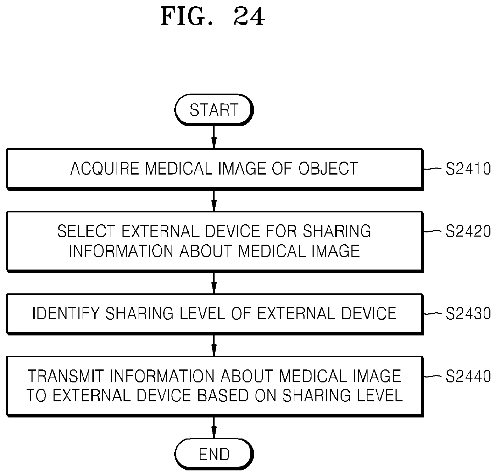

In the above description, the method of sharing information about ultrasound images between the ultrasound apparatus 3000 and at least one external device according to a sharing level is described as an example; however, one or more examples of the present disclosure are not limited thereto. For example, other medical imaging apparatuses in addition to the ultrasound apparatus 3000 may share information about medical images with the external device according to a sharing level of the external device. A method of sharing information about medical images by the medical imaging apparatus with at least one external device will be described with reference to FIG. 24 .

FIG. 24 is a flowchart illustrating an information sharing method of a medical imaging apparatus according to an example of the present disclosure.

In operation S2410, the medical imaging apparatus may acquire a medical image of an

object. According to the present example, the medical imaging apparatus may directly generate the medical image or may receive the medical image from outside.

In the present specification, the medical imaging apparatus may include a magnetic resonant imaging (MRI) apparatus, a computerized tomography (CT) apparatus, an X-ray photographing apparatus, an angiography apparatus, and the like; however, one or more examples of the present disclosure are not limited thereto.

An MRI apparatus is an apparatus for acquiring a sectional image of a part of an object by expressing, in a contrast comparison, a strength of a MR signal with respect to a radio frequency (RF) signal generated in a magnetic field having a specific strength.

Since a CT apparatus is capable of providing a cross-sectional image of a object, the CT apparatus may express an inner structure (e.g., an organ such as a kidney, a lung, etc.) of the object without an overlap therebetween, compared to a general X-ray photographing apparatus. The CT apparatus may obtain a plurality of pieces of image data with a thickness not more than 2mm for several tens to several hundreds of times per second and then may process the plurality of pieces of image data, so that the CT apparatus may provide a relatively accurate a cross-sectional image of the object.

An X-ray photographing apparatus is an apparatus of imaging internal organic structure of an object by transmitting an X-ray through the object. An angiography apparatus is an apparatus for imaging blood vessels (arteries and veins), in which a contrast medium is injected through a thin tube having a diameter of about 2 mm, called as a catheter, by transmitting an X-ray.

According to the present example, the medical imaging apparatus may be realized in various formats. For example, the medical imaging apparatus recited in the present specification may be configured as a mobile terminal type, as well as a fixed terminal type. Examples of the mobile terminal may include smartphones, laptop computers, personal digital assistants (PDAs), tablet PCs, and the like.

According to the examples of the present disclosure, the medical image may include an MRI, a CT image, an ultrasound image, and an X-ray image; however, the examples of the present disclosure are not limited thereto. Also, the medical image may be a two-dimensional image, a three-dimensional image, or a four-dimensional image; however, the examples of the present disclosure are not limited thereto. Hereinafter, the medical image is assumed as a two-dimensional X-ray image for the convenience of description.

In operation S2420, the medical imaging apparatus may select an external device, with which information about a medical image will be shared.

According to the present example, the medical imaging apparatus may select a first device 1000 and a second device 2000 that are connected to the medical imaging apparatus based on system settings.

According to another example of the present disclosure, the medical imaging apparatus may select the first device 1000 and the second device 2000 based on a user input. For example, the medical imaging apparatus may provide a device list including identification information of devices that may communicate with the medical imaging apparatus on a screen. The medical imaging apparatus may receive a user input for the first device 1000 and the second device 2000 in the device list. The user of the medical imaging apparatus according to the present example may be a sonographer, a radiological technologist, an ambulance attendant, or an ordinary person; however, the examples of the present disclosure are not limited thereto.

Information about the medical image may include at least one piece of information from among information about the medical image itself, annotation information for describing all or some of the medical image, identification mark information for identifying a region of interest in the medical image, analyzing information generated by the radiological technologist or the sonographer, information about the object, and measuring information; however, the examples of the present disclosure are not limited thereto.

In operation S2430, the medical imaging apparatus may identify a sharing level of the selected external device. The sharing level may include information representing authority of a user of the external device for checking the information about the medical image. The sharing level may be set in advance by an information sharing system or a user.

For example, the medical imaging apparatus may identify information about a first sharing level corresponding to identification information of the first device 1000 and/or a second sharing level corresponding to the identification information of the second device 2000.

According to the present example, the medical imaging apparatus may receive information about the first sharing level and/or information about the second sharing level from a server 4000. Also, according to another example of the present disclosure, the medical imaging apparatus may read the information about the first sharing level and/or the information about the second sharing level stored in the memory.

According to the example of the present disclosure, the first sharing level of the first device 1000 and the second sharing level of the second device 2000 may be different from each other. For example, if the first device 1000 is an object device and the second device 2000 is a medical expert device, the sharing level of the first device 1000, that is, the object device, may be lower than the sharing level of the second device 2000, that is, the medical expert device. Relative low sharing level denotes that kind and/or amount of information that may be shared is relatively small.

In operation S2440, the medical imaging apparatus may transmit information about the medical image to the external device based on the sharing level.

According to the example of the present disclosure, the medical imaging apparatus may transmit first medical image information corresponding to the first sharing level to the first device 1000, or may transmit second medical image information corresponding to the second sharing level to the second device 2000. Here, according to the present example, the medical imaging apparatus may generate the first medical image information and the second medical image information.

The medical imaging apparatus may generate the first medical image information corresponding to the first sharing level of the first device 1000. For example, if the first device 1000 is the object device, the medical imaging apparatus may generate the first medical image information including the medical image, annotation for describing the medical image, an identification mark on the region of interest, and measuring information according to the first sharing level of the object device.

The medical imaging apparatus may generate the second medical image information corresponding to the second sharing level of the second device 2000. For example, if the second device 2000 is the medical expert device, the medical imaging apparatus may generate the second medical image information including the medical image, measuring information of the medical image, analyzing information of the medical image by the radiological technologist, and object information according to the second sharing level of the medical expert device.

Therefore, according to the example of the present disclosure, in a case where the first device 1000 is the object device and the second device 2000 is the medical expert device, the medical imaging apparatus may share the first medical image information including the annotation for describing the medical image, the identification mark on the region of interest, and the measuring information with the first device 1000, and may share the second medical image information including the medical image, the measuring information of the medical image, the analyzing information of the medical image by the radiological technologist, and the object information with the second device 2000.

In addition, according to the example of the present disclosure, the medical imaging apparatus encodes the first medical image information and/or the second medical image information with an encoding code that is negotiated in advance or set in advance for security of the first medical image information and/or the second medical image information, and transmits the encoded first medical image information and/or second medical image information to the first device 1000 or the second device 2000.

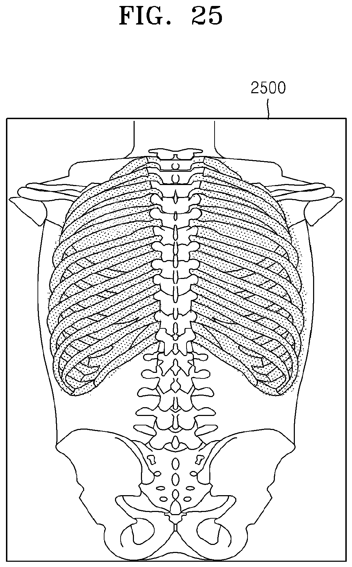

Hereinafter, processes of sharing information about the medical image (for example, an X-ray image) by the medical imaging apparatus (for example, an X-ray photographing apparatus) with at least one external device according to the sharing level will be described below with reference to FIGS. 25 through 27 .

FIG. 25 is a diagram showing an X-ray image acquired by an X-ray photographing apparatus according to an example of the present disclosure.

As shown in FIG. 25 , the X-ray photographing apparatus may acquire an X-ray chest image 2500 of an object by using the X-ray. In this case, the X-ray photographing apparatus may select an external device with which information about the X-ray chest image 2500 will be shared. For example, the X-ray photographing apparatus may select an object device for describing the X-ray chest image 2500 to the object.

The X-ray photographing apparatus may identify a sharing level of the selected object device. In addition, the X-ray photographing apparatus may generate information about the X-ray chest image 2500 that will be shared with the object device according to the sharing level.

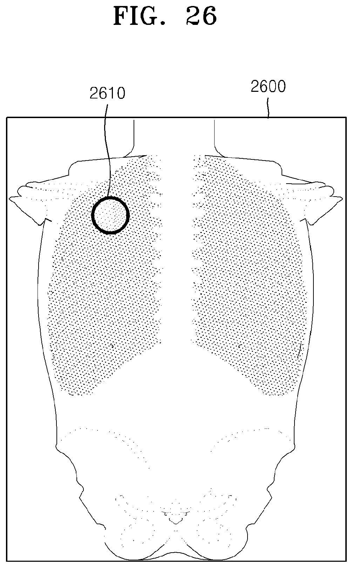

For example, if the sharing level of the object device is a first level, the X-ray photographing apparatus generates a bone suppression image that is obtained by deleting bones from the X-ray chest image 2500, and marks a region of interest on the bone suppression image. This will be described with reference to FIG. 26 .

As shown in FIG. 26 , the X-ray photographing apparatus may generate a bone suppression image 2600 by deleting bones from the X-ray chest image 2500 so that the object may clearly see lesion. In addition, the X-ray photographing apparatus selects a region that is suspected to have nodule on the bone suppression image 2600 as a region of interest 2610, and displays a mark for the region of interest 2610. Here, the X-ray photographing apparatus may select the region of interest 2610 automatically or based on a user input. In FIG. 26 , the region of interest 2610 is marked by a circle; however, the examples of the present disclosure are not limited thereto.

The X-ray photographing apparatus may transmit information about the bone suppression image 2600 and information about the region of interest 2610 to the object device according to the sharing level of the object device (for example, the first level). Here, the object device may display the bone suppression image 2600 including the region of interest 2610 on a screen.

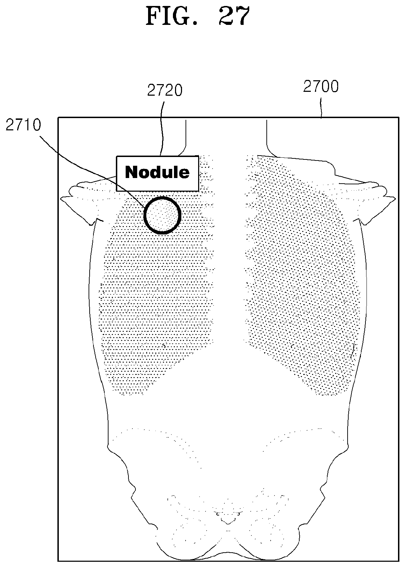

In addition, if the sharing level of the object device is a second level, the X-ray photographing apparatus may additionally generate information about annotation for describing the region of interest 2610 (for example, text, font, and display location information). This will be described with reference to FIG. 27 .

As shown in FIG. 27 , the X-ray photographing apparatus may generate a bone suppression image 2700 by deleting bones from the X-ray chest image 2500 so that the object may clearly see the lesion. In addition, the X-ray photographing apparatus selects a part suspected to be a nodule as a region of interest 2710 on the bone suppression image 2700, and may generate an annotation 2720 about the region of interest 2710. The X-ray photographing apparatus may generate the annotation 2720 automatically or based on a user input.

The X-ray photographing apparatus may transmit information about the bone suppression image 2700, information about the region of interest 2710, and information about the annotation 2720 to the object device according to the sharing level of the object device (for example, the second level). The object device may display the bone suppression image 2700 including the region of interest 2710 and the annotation 2720 on a screen.

## Claims

1. An ultrasound apparatus (3000) comprising:
an ultrasound image obtaining unit (3100), a communication unit (3200), a display unit (3600), and a control unit (3300);
wherein the ultrasound image obtaining unit is configured to acquire ultrasound image data about an object, wherein the ultrasound image data may be 2D ultrasound image data or 3D ultrasound image data about the object;
the communication unit includes one or more components enabling communications between the ultrasound apparatus and a first device (1000) and between the ultrasound apparatus and a second device (2000), and is configured to transmit first ultrasound information about an ultrasound image to the first device (1000) and second ultrasound information about the ultrasound image to the second device according to sharing levels that are set in advance;
the display unit is configured to display and output information processed by the ultrasound apparatus;
and the control unit is configured to control the overall operation of the ultrasound apparatus;
wherein the communication unit is further configured to receive control information from the second device (2000), wherein the control information includes a control command for selecting and displaying a different ultrasound image from a currently displayed image, and a control command for expanding or reducing the ultrasound image;
and wherein the control unit is further configured to execute a control command corresponding to the control information transmitted from the second device (2000).

2. The ultrasound image apparatus according to claim 1, wherein the control information further includes a control command for storing the ultrasound image, a control command for 3D rendering the ultrasound image, a control command for adding an annotation or a body marker, a control command for measuring a region of interest, and a control command for correcting analysis information of the ultrasound image.

3. The ultrasound apparatus according to claim 1, further comprising a user input unit (3400) in the form of a touch pad.

4. The ultrasound apparatus according to claim 3, wherein a display panel and the touch pad are layered to form a touch screen, so that the display unit (3600) is configured to be used as an input device as well as an output device.

5. The ultrasound apparatus according to claim 4, wherein the ultrasound apparatus is configured to sense a touch gesture of a user on an ultrasound image via the touch screen, wherein the touch gesture includes at least one of a tap, a touch and hold, a double tap, a drag, panning, a flick, a drag and drop, a swipe, or a pinch.

6. The ultrasound apparatus according to claim 1, wherein the ultrasound apparatus is configured to display, via the display unit, at least one ultrasound image, and to display a user interface, UI, or graphical user interface, GUI, related to a control panel.

7. A second device (2000), the second device being a medical expert device, comprising:
a display unit (210), a user input unit (220), a communication unit (230), and a control unit (240);
wherein the display unit is configured to display and output information processed by the medical expert device;
the communication unit includes one or more components enabling communications between an ultrasound apparatus (3000) and the second device (2000), and is configured to receive second ultrasound information about an ultrasound image from the ultrasound apparatus according to a sharing level that is set in advance;
wherein the communication unit is further configured to transmit control information for controlling
the ultrasound apparatus (3000) to the ultrasound apparatus (3000),
wherein the control information includes a control command for selecting and displaying another ultrasound image, and a control command for expanding or reducing the ultrasound image.

8. The second device according to claim 7, wherein the control information further includes control command for storing the ultrasound image, a control command for 3D rendering the ultrasound image, a control command for adding an annotation or a body marker, a control command for measuring a region of interest, and a control command for correcting analysis information of the ultrasound image.

9. The second device according to claim 7, wherein the input unit (220) is a touch pad.

10. An information sharing system,
including an ultrasound apparatus (3000) according to any of claims 1 to 6, and a second device (2000) according to any of claims 7 to 9.

## Patentansprüche

1. Ultraschallvorrichtung (3000), umfassend:
eine Ultraschallbildgewinnungseinheit (3100), eine Kommunikationseinheit (3200), eine Anzeigeeinheit (3600) und eine Steuereinheit (3300);
wobei die Ultraschallbildgewinnungseinheit dazu konfiguriert ist, Ultraschallbilddaten über ein Objekt zu erfassen, wobei die Ultraschallbilddaten 2D-Ultraschallbilddaten oder 3D-Ultraschallbilddaten über das Objekt sein können;
die Kommunikationseinheit eine oder mehrere Komponenten beinhaltet, die Kommunikationen zwischen der Ultraschallvorrichtung und einer ersten Vorrichtung (1000) und zwischen der Ultraschallvorrichtung und einer zweiten Vorrichtung (2000) ermöglichen, und dazu konfiguriert ist, erste Ultraschallinformationen über ein Ultraschallbild an die erste Vorrichtung (1000) und zweite Ultraschallinformationen über das Ultraschallbild an die zweite Vorrichtung gemäß Freigabestufen, die im Voraus festgelegt sind, zu übertragen;
die Anzeigeeinheit dazu konfiguriert ist, durch die Ultraschallvorrichtung verarbeitete Informationen anzuzeigen und auszugeben;
und die Steuereinheit dazu konfiguriert ist, den Gesamtbetrieb der Ultraschallvorrichtung zu steuern;
wobei die Kommunikationseinheit ferner dazu konfiguriert ist, Steuerinformationen von der zweiten Vorrichtung (2000) zu empfangen, wobei die Steuerinformationen einen Steuerbefehl zum Auswählen und Anzeigen eines von einem aktuell angezeigten Bild verschiedenen Ultraschallbildes und einen Steuerbefehl zum Vergrößern oder Verkleinern des Ultraschallbildes beinhalten;
und wobei die Steuereinheit ferner dazu konfiguriert ist, einen Steuerbefehl auszuführen, der den von der zweiten Vorrichtung (2000) übertragenen Steuerinformationen entspricht.

2. Ultraschallbildvorrichtung nach Anspruch 1, wobei die Steuerinformationen ferner einen Steuerbefehl zum Speichern des Ultraschallbildes, einen Steuerbefehl zum 3D-Rendern des Ultraschallbildes, einen Steuerbefehl zum Hinzufügen einer Anmerkung oder einer Körpermarkierung, einen Steuerbefehl zum Messen einer Region von Interesse und einen Steuerbefehl zum Korrigieren von Analyseinformationen des Ultraschallbildes beinhalten.

3. Ultraschallvorrichtung nach Anspruch 1, ferner umfassend eine Benutzereingabeeinheit (3400) in Form eines Touchpads.

4. Ultraschallvorrichtung nach Anspruch 3, wobei eine Anzeigetafel und das Touchpad geschichtet sind, um einen Touchscreen zu bilden, sodass die Anzeigeeinheit (3600) dazu konfiguriert ist, sowohl als Eingabevorrichtung als auch als Ausgabevorrichtung verwendet zu werden.

5. Ultraschallvorrichtung nach Anspruch 4, wobei die Ultraschallvorrichtung dazu konfiguriert ist, eine Berührungsgeste eines Benutzers auf einem Ultraschallbild über den Touchscreen zu erfassen, wobei die Berührungsgeste mindestens eines von einem Antippen, einem Berühren und Halten, einem doppelten Antippen, einem Ziehen, einem Schwenken, einem Schnipsen, einem Ziehen und Ablegen, einem Wischen oder einem Zusammenziehen beinhaltet.

6. Ultraschallvorrichtung nach Anspruch 1, wobei die Ultraschallvorrichtung dazu konfiguriert ist, über die Anzeigeeinheit mindestens ein Ultraschallbild anzuzeigen und eine Benutzerschnittstelle, UI, oder eine grafische Benutzerschnittstelle, GUI, in Bezug auf eine Steuertafel anzuzeigen.

7. Zweite Vorrichtung (2000), wobei die zweite Vorrichtung eine medizinische Expertenvorrichtung ist, umfassend:
eine Anzeigeeinheit (210), eine Benutzereingabeeinheit (220), eine Kommunikationseinheit (230) und eine Steuereinheit (240);
wobei die Anzeigeeinheit dazu konfiguriert ist, durch die medizinische Expertenvorrichtung verarbeitete Informationen anzuzeigen und auszugeben;
die Kommunikationseinheit eine oder mehrere Komponenten beinhaltet, die Kommunikationen zwischen einer Ultraschallvorrichtung (3000) und der zweiten Vorrichtung (2000) ermöglichen, und dazu konfiguriert ist, zweite Ultraschallinformationen über ein Ultraschallbild von der Ultraschallvorrichtung gemäß einer Freigabestufe, die im Voraus festgelegt ist, zu empfangen;
wobei die Kommunikationseinheit ferner dazu konfiguriert ist, Steuerinformationen zum Steuern
der Ultraschallvorrichtung (3000) an die Ultraschallvorrichtung (3000) zu übertragen,
wobei die Steuerinformationen einen Steuerbefehl zum Auswählen und Anzeigen eines weiteren Ultraschallbildes und einen Steuerbefehl zum Vergrößern oder Verkleinern des Ultraschallbildes beinhalten.

8. Zweite Vorrichtung nach Anspruch 7, wobei die Steuerinformationen ferner einen Steuerbefehl zum Speichern des Ultraschallbildes, einen Steuerbefehl zum 3D-Rendern des Ultraschallbildes, einen Steuerbefehl zum Hinzufügen einer Anmerkung oder einer Körpermarkierung, einen Steuerbefehl zum Messen einer Region von Interesse und einen Steuerbefehl zum Korrigieren von Analyseinformationen des Ultraschallbildes beinhalten.

9. Zweite Vorrichtung nach Anspruch 7, wobei die Eingabeeinheit (220) ein Touchpad ist.

10. Informationsfreigabesystem,
umfassend eine Ultraschallvorrichtung (3000) nach einem der Ansprüche 1 bis 6 und eine zweite Vorrichtung (2000) nach einem der Ansprüche 7 bis 9.

## Revendications

1. Dispositif ultrasonore (3000), comprenant :
une unité d'obtention d'image ultrasonore (3100), une unité de communication (3200), une unité d'affichage (3600) et une unité de commande (3300) ;
dans lequel l'unité d'obtention d'image ultrasonore est configurée pour acquérir des données d'image ultrasonore concernant un objet, dans lequel les données d'image ultrasonore peuvent être des données d'image ultrasonore 2D ou des données d'image ultrasonore 3D concernant l'objet ;
l'unité de communication comprend un ou plusieurs composants permettant des communications entre le dispositif ultrasonore et un premier dispositif (1000) et entre le dispositif ultrasonore et un deuxième dispositif (2000), et est configurée pour transmettre des premières informations ultrasonores concernant une image ultrasonore au premier dispositif (1000) et des deuxièmes informations ultrasonores concernant l'image ultrasonore au deuxième dispositif selon des niveaux de partage qui sont définis à l'avance ;
l'unité d'affichage est configurée pour afficher et délivrer en sortie des informations traitées par le dispositif ultrasonore ;
et l'unité de commande est configurée pour commander le fonctionnement global du dispositif ultrasonore ;
dans lequel l'unité de communication est en outre configurée pour recevoir des informations de commande en provenance du deuxième dispositif (2000), dans lequel les informations de commande comprennent une instruction de commande pour sélectionner et afficher une image ultrasonore différente d'une image actuellement affichée, et une instruction de commande pour agrandir ou réduire l'image ultrasonore ;
et dans lequel l'unité de commande est en outre configurée pour exécuter une instruction de commande correspondant aux informations de commande transmises depuis le deuxième dispositif (2000).

2. Dispositif d'image ultrasonore selon la revendication 1, dans lequel les informations de commande comprennent en outre une instruction de commande pour stocker l'image ultrasonore, une instruction de commande pour un rendu 3D de l'image ultrasonore, une instruction de commande pour ajouter une annotation ou un marqueur corporel, une instruction de commande pour mesurer une région d'intérêt et une instruction de commande pour corriger des informations d'analyse de l'image ultrasonore.

3. Dispositif ultrasonore selon la revendication 1, comprenant en outre une unité d'entrée d'utilisateur (3400) sous la forme d'un pavé tactile.

4. Dispositif ultrasonore selon la revendication 3, dans lequel un panneau d'affichage et le pavé tactile sont superposés en couches pour former un écran tactile, de sorte que l'unité d'affichage (3600) est configurée pour être utilisée en tant que dispositif d'entrée ainsi qu'en tant que dispositif de sortie.

5. Dispositif ultrasonore selon la revendication 4, dans lequel le dispositif ultrasonore est configuré pour détecter un geste tactile d'un utilisateur sur une image ultrasonore par l'intermédiaire de l'écran tactile, dans lequel le geste tactile comprend au moins l'un d'un tapotement, d'un toucher maintenu, d'un double tapotement, d'un traînage, d'un panoramique, d'un effleurement, d'un glisser-déposer, d'un balayage ou d'un pincement.

6. Dispositif ultrasonore selon la revendication 1, dans lequel le dispositif ultrasonore est configuré pour afficher, par l'intermédiaire de l'unité d'affichage, au moins une image ultrasonore, et pour afficher une interface utilisateur, UI, ou une interface utilisateur graphique, GUI, relative à un panneau de commande.

7. Deuxième dispositif (2000), le deuxième dispositif étant un dispositif d'expert médical, comprenant :
une unité d'affichage (210), une unité d'entrée d'utilisateur (220), une unité de communication (230) et une unité de commande (240) ;
dans lequel l'unité d'affichage est configurée pour afficher et délivrer en sortie des informations traitées par le dispositif d'expert médical ;
l'unité de communication comprend un ou plusieurs composants permettant des communications entre un dispositif ultrasonore (3000) et le deuxième dispositif (2000), et est configurée pour recevoir des deuxièmes informations ultrasonores concernant une image ultrasonore en provenance du dispositif ultrasonore selon un niveau de partage qui est défini à l'avance ;
dans lequel l'unité de communication est en outre configurée pour transmettre des informations de commande pour commander
le dispositif ultrasonore (3000) au dispositif ultrasonore (3000),
dans lequel les informations de commande comprennent une instruction de commande pour sélectionner et afficher une autre image ultrasonore, et une instruction de commande pour agrandir ou réduire l'image ultrasonore.

8. Deuxième dispositif selon la revendication 7, dans lequel les informations de commande comprennent en outre une instruction de commande pour stocker l'image ultrasonore, une instruction de commande pour un rendu 3D de l'image ultrasonore, une instruction de commande pour ajouter une annotation ou un marqueur corporel, une instruction de commande pour mesurer une région d'intérêt et une instruction de commande pour corriger des informations d'analyse de l'image ultrasonore.

9. Deuxième dispositif selon la revendication 7, dans lequel l'unité d'entrée (220) est un pavé tactile.

10. Système de partage d'informations,
comprenant un dispositif ultrasonore (3000) selon l'une quelconque des revendications 1 à 6, et un deuxième dispositif (2000) selon l'une quelconque des revendications 7 à 9.
